# EUROPÄISCHE PATENTSCHRIFT

(11) **EP 1 439 879 B1**
(45) Veröffentlichungstag und Bekanntmachung des Hinweises auf die Patenterteilung: **14.07.2010**
(21) Anmeldenummer: 02774740.1
(22) Anmeldetag: 22.10.2002
(51) Int. Cl.: A61M 16/06

(54) **Medizinische Maske**
Medical Mask
Masque médical

(30) Priorität: 22.10.2001 DE 10151984; 17.01.2002 DE 10201682; 14.03.2002 WO PCT/EP02/02877
(43) Veröffentlichungstag der Anmeldung: 28.07.2004
(73) Patentinhaber: MAP Medizin-Technologie GmbH, 82152 Martinsried (DE)
(72) Erfinder: LANG, Bernd, 82166 Gräfelfing (DE); BIENER, Achim, 85445 Aufkirchen (DE); HEIDMANN, Dieter, 82538 Geretsried (DE); VÖGELE, Harald, 82131 Gauting (DE); MADAUS, Stefan, 82152 Krailling (DE)
(74) Vertreter: Heunemann, Dieter
(86) Internationale Anmeldenummer: PCT/EP2002/011798
(87) Internationale Veröffentlichungsnummer: WO 2003/035156

(56) Entgegenhaltungen:
- WO-A-01/97892
- WO-A-99/58181
- WO-A-00/069521
- DE-C- 284 800

## Beschreibung

### Gebiet der Erfindung

Die Erfindung betrifft eine Atemmaskenanordnung wie sie beispielsweise im Rahmen einer CPAP-Therapie zur Behandlung schlafbezogener Atmungsstörungen Anwendung finden kann.

### Hintergrund der Erfindung

Im Rahmen der genannten CPAP-Therapie kann einem Patienten über eine Atemmaskenanordnung ein atembares Gas, insbesondere Umgebungsluft auf einem Druckniveau zugeführt werden das über dem Umgebungsdruckniveau liegt. Durch das unter Druck stehende Atemgas kann eine pneumatische Schienung der oberen Atemwege erreicht werden und hierdurch etwaigen Obstruktionen vorgebeugt werden. Im Rahmen der Durchführung einer Druckbeatmungs- bzw. CPAP-Therapie werden die zur Zufuhr des Atemgases erforderlichen Atemmaskenanordnungen üblicherweise über die gesamte Schlaf- oder Ruhephase des Patienten hinweg von diesem getragen. Die Atemmaskenanordnung stützt sich üblicherweise über eine Dichtlippenzone im Umgebungsbereich der Nase des Maskenanwenders sowie über eine Stirnauflageeinrichtung im Stirnbereich des Maskenanwenders ab. Die zur Applikation der Atemmaskenanordnung erforderlichen Haltekräfte können durch eine Fixiereinrichtung die zum Beispiel ein um den Hinterkopf des Maskenanwenders herumgeführte Kopfband aufweist, aufgebracht werden. Im Bereich der Auflagezone der Dichtlippeneinrichtung sowie im Auflagebereich der Stirnauflageeinrichtung können unter Umständen Flächenpressungen auftreten, die zu einer erheblichen Beeinträchtigung des Tragekomforts der Atemmaskenanordnung führen. In Abhängigkeit von der individuellen Gesichtstektur des Maskenanwenders sind teilweise erhebliche Maskenanpreßkräfte erforderlich, um die gewünschte Dichtwirkung zu erreichen. Im Bereich der Auflagezonen der Atemmaske auf dem Gesicht des Patienten können hierbei in unangenehmer Weise deutlich sichtbare Druckstellen auch im Stirnbereich verursacht werden.

WO 00/69521 betrifft eine nasale Maskenanordnung, die eine Manschette und ein Dichtelement umfasst, wobei die Manschette eine Mehrzahl von Durchbrüchen aufweist, an welchen Kopfbänder befestigt sind. Zudem weist das Dichtelement eine Lippe, einen Aufnahmehohlraum und einen Halsteil auf, wobei der Halsteil des Dichtelements mit der Manschette verbunden ist. Zur Applikation der nasalen Maskenanordnung auf der Nase eines Patienten wird die Lippe über den Aufnahmehohlraum und über den Halsteil auf das Gesicht des Patienten gedrückt, indem die Manschette mittels der Kopfbänder am Kopf des Patienten befestigt wird.

DE 284800 betrifft eine Inhalationsmaske, die am Kopf des Patienten durch ein biegsames und elastisches Band befestigt wird, welches sich von der Vorderseite des Kopfes bis zur Rückseite desselben erstreckt. Dieses Band ist mit pneumatischen Polstern versehen, welche an gegenüberliegenden Enden von quer verlaufenden Platten angebracht sind, von denen eine unmittelbar am vorderen Ende des Bandes befestigt ist, während die andere Platte mit einem weiteren Band angebracht ist, welches mit dem ersten Band verschiebbar in Verbindung steht. An dem Kopfband ist ein weiteres, federndes Band befestigt, welches zwischen den Enden des Kopfbandes angebracht ist und an dessen vorderem Ende durch eine Öffnung desselben ein mit Flanschen versehener Bolzen ragt, an dem wiederum einstellbare Arme schwingbar gelagert sind. An den Enden der Arme ist eine Nasenklappe befestigt.

WO 99/58 181 betrifft eine Atemmasken anordnung mit einem sattelförmigen Nasenteil das von einem federnden Luftzulauf-Schlauch gestützt wird.

### Aufgabe der Erfindung

Der Erfindung liegt die Aufgabe zugrunde, Lösungen bereitzustellen durch welche eine Atemmaskenanordnung in einer korrekten Applikationsposition fixiert werden kann und ein hoher Tragekomfort erreichbar ist.

### Erfindungsgemäße Lösung

Diese Aufgabe wird erfindungsgemäß gelöst, durch eine Atemmaskenanordnung wie in Anspruch 1 definiert mit einem Gewölbekörper, einer Dichtlippeneinrichtung zur Auflage auf der Gesichtsfläche eines Maskenanwenders, einer Atemgasleitungseinrichtung zur Zuleitung von Atemgas zu einem von dem Gewölbekörper begrenzten und mit der Nasen- und/oder Mundöffnung des Maskenanwenders in Verbindung stehendem Maskeninnenraum und einer Applikationsstruktur zur Applikation der Dichtlippeneinrichtung gemeinsam mit dem Gewölbekörper, wobei die Applikationsstruktur einen Trägerabschnitt aufweist an welchem ein Atemgasleitungsorgan Lösbar angekoppelt ist.

Dadurch wird es auf vorteilhafte Weise möglich, eine robust aufgebaute und in vorteilhafter Weise reinigbare Atemmaskenanordnung zu schaffen, die sich durch eine hohe Dichtigkeit auszeichnet.

Gemäß einer besonders bevorzugten Ausführungsform der Erfindung ist das Atemgasleitungsorgan als Rohrstutzen ausgebildet. Ein derartiger Rohrstutzen ist vorzugsweise aus einem spülmaschinenfesten Kunststoffmaterial gefertigt, so dass dieses Atemgasleitungsorgan bei vergleichsweise hohen Temperaturen gereinigt und dabei sterilisiert werden kann. Der Rohrstutzen ist vorzugsweise derart ausgebildet, dass dessen Innendurchmesser im Bereich von 12 bis 34 mm liegt. Der Rohrstutzen kann einen, im wesentlichen kreisförmigen oder vorzugsweise einen polygonalen Querschnitt aufweisen. Der Rohrstutzen kann als Leitungskrümmer ausgebildet sein, durch welchen eine leichte Umlenkung des Atemgasstromes um einen Winkel im Bereich von 0 bis 45° erfolgt.

Das insbesondere als Rohrstutzen ausgebildete Leitungsorgan ist vorzugsweise lösbar an dem Trägerabschnitt anbringbar. Zur Ankoppelung des Atemgasleitungsorganes an den Trägerabschnitt ist vorzugsweise eine Verrastungseinrichtung vorgesehen. Diese Verrastungseinrichtung ist gemäß einem besonderen Aspekt der vorliegenden Erfindung in vorteilhafter Weise als Bajonett- bzw. Drehverrastungseinrichtung ausgebildet.

Die Applikationsstruktur der Atemmaskenanordnung umfasst in vorteilhafter Weise einen Rahmenabschnitt der mit der Dichtlippeneinrichtung und/oder mit dem Gewölbekörper lösbar koppelbar ist. Vorzugsweise ist der Rahmenabschnitt derart ausgebildet, dass dieser den Gewölbekörper ring- oder bügelartig umfasst. Der Trägerabschnitt ist in vorteilhafter Weise aus einem Kunststoffmaterial gefertigt und mit Halteorganen versehen, über welche der Trägerabschnitt beispielsweise mit einer unteren Gurtbandanordnung eines Kopfbandes, koppelbar ist. In vorteilhafter Weise sind diese Halteorgane als Haltebügel ausgebildet, durch welche ein Endabschnitt der genannten unteren Gurtbandanordnung hindurchführbar ist. Diese Haltebügel sind vorzugsweise integral mit dem Trägerabschnitt ausgebildet. Die Innenumfangswandung einer von den Haltebügeln gebildeten Durchführungsöffnung, weist vorzugsweise einen Verlauf auf, der hinsichtlich des Formwerkzeuges eine schieberfreie Entformung der Haltebügel gestattet.

Der zur Fixierung des Atemgasleitungsorganes vorgesehene Trägerabschnitt ist in vorteilhafter Weise integral mit dem Rahmenabschnitt ausgebildet. Der Trägerabschnitt ist hierbei mit Vorteil derart ausgebildet, dass dieser eine Einsatzöffnung aufweist, in die das Atemgasleitungsorgan lösbar einsetzbar ist. Der Trägerabschnitt ist vorzugsweise derart angeordnet, dass dieser im wesentlichen senkrecht, zu einer durch den Rahmenabschnitt definierten Rahmenfläche verläuft.

Im Bereich der Einsatzöffnung bildet der Trägerabschnitt vorzugsweise jene mit dem als Rohrstutzen ausgebildeten Atemgasleitungsorgan in Eingriff bringbare Koppelungsstrukturen.

Der Gewölbekörper weist vorzugsweise einen Koppelungsabschnitt auf, über welchen der Gewölbekörper in abdichtender Weise mit dem Atemgasleitungsorgan verbindbar ist. Vorzugsweise ist der Gewölbekörper aus einem Elastomermaterial gefertigt und unter elastischer Aufweitung auf einen, durch die Einsatzöffnung hindurchgeführten und zur Dichtlippeneinrichtung vordringenden Abschnitt des Rohrstutzens aufgesetzt. An diesem zur Dichtlippeneinrichtung vordringenden Abschnitt des Rohrstutzens ist vorzugsweise ein Umfangswulst ausgebildet, durch welchen der Gewölbekörper und der Rohrstutzen auf zuverlässige Weise in Fügestellung gehalten werden.

Gemäß einer besonders bevorzugten Ausführungsform der Erfindung ist der Gewölbekörper integral mit der Dichtlippeneinrichtung ausgebildet. Dadurch wird es auf vorteilhafte Weise möglich, Fugen bzw. Spalten im Übergangsbereich zwischen Dichtlippeneinrichtung und Gewölbekörper zu vermeiden. Darüber hinaus wird es auf vorteilhafte Weise möglich, die Dichtlippeneinrichtung und den Gewölbekörper als elastomeres Integralteil in den Rahmenabschnitt einzusetzen.

Eine besonders zuverlässige Ableitung von CO₂ befrachtetem Atemgas an die Umgebung wird gemäß einer besonders bevorzugten Ausführungsform der Erfindung dadurch erreicht, dass der Gewölbekörper mit Öffnungen versehen ist, durch welche das im Innenbereich des Gewölbekörpers unter Druck stehende Atemgas zur Umgebung hin entweichen kann. Die Öffnungen sind vorzugsweise derart ausgebildet, dass sich deren Querschnitt in Austrittsöffnung erweitert. Diese Austrittsöffnungen sind vorzugsweise derart angeordnet, dass sich diese möglichst nahe in dem, in Applikationsposition der Atemmaskenanordnung den Nasenöffnungen eines Maskenanwenders benachbarten Bereich befinden.

Gemäß einer Auführungsform umfasst die Applikationsstruktur eine Stirnabstützeinrichtung zur Abstützung der Atemmaskenanordnung im Stirnbereich des Maskenanwenders. Die Stirnabstützeinrichtung ist in vorteilhafter Weise über eine Gelenkvorrichtung mit dem den Gewölbekörper umgreifenden Rahmenabschnitt verbunden. Dadurch wird es auf vorteilhafte Weise möglich, die Position der Stirnabsttitzeinrichtung an die individuelle Gesichtstektur des Maskenanwenders anzupassen. Vorzugsweise umfasst die Gelenkeinrichtung eine Bogenbahnführung, durch welche die Stirnabstützeinrichtung veränderbar positionierbar ist.

Das vorzugsweise lösbar, mit dem Trägerabschnitt koppelbare Atemgasleitungsorgan, bildet in vorteilhafter Weise einen Dockingport der beispielsweise auch Teil einer Dreh-Koppelungsstruktur bilden kann. An dem Dockingport können weitere Anschlusseinrichtungen, insbesondere kleinere Rohrstutzen ausgebildet sein, über welche beispielsweise ein Druckmessschlauch an die Atemmaskenanordnung angekoppelt werden kann oder gegebenenfalls auch eine zusätzliche Sauerstoffzufuhr erfolgen kann.

Die Stirnabstützeinrichtung ist vorzugsweise aus einem thermoplastischen Kunststoffmaterial gefertigt und mit einer Stirnpolstereinrichtung versehen. Die Stirnpolstereinrichtung ist vorzugsweise durch pad-artig ausgebildete Elastomerelemente gebildet, die über eine Steckverbindungsstruktur mit dem vorzugsweise bügelartig ausgebildeten Aufnahmeabschnitt der Stirnabstützeinrichtung koppelbar sind. Die Stirnabstützeinrichtung ist dabei vorzugsweise derart ausgebildet, dass die Elastomerelemente an unterschiedlichen Stellen an der Stirnabstützeinrichtung anbringbar sind. Vorzugsweise sind die Elastomerelemente weiterhin derart ausgebildet, dass durch die Art der Befestigung derselben an dem Bügelteil ebenfalls unterschiedliche Positionen der Auflagebereiche der Elastomerelemente auf der Stirn des Maskenanwenders erreichbar sind. Die Elastomerelemente sind vorzugsweise aus einem Silikonkautschukmaterial gefertigt und im Bereich ihrer Auflagefläche derart geformt, dass die Übertragung der Aulagegekräfte auf die Stirnfläche des Maskenanwenders unter einer physiologisch gut verträglichen Flächenpressung erfolgt. Dies kann insbesondere dadurch erreicht werden, dass die Elastomerelemente auf einer ihrer Auflageseite abgewandten Rückseite mit einem exzentrisch angeordneten Standfuß versehen sind, der eine Kippbewegung des am Maskenanwender aufsitzenden Auflageabschnitts zulässt.

An dem Bügelteil sind vorzugsweise auch Koppelungsabschnitte vorgesehen, welche eine Koppelung der Stirnabstützeinrichtung mit einer oberen Stirnbandanordnung eines Kopfbandes ermöglicht. Diese Koppelungsabschnitte können als Bandlasche ausgebildet sein. Es ist auch möglich, die Stirnabstützeinrichtung, beispielsweise über eine Klettverschlusseinrichtung an einer vorzugsweise gepolsterten Stirnbandanordnung zu befestigen.

Gemäß einer Ausführungsform der Erfindung umfaßt eine Stirnauflageeinrichtung mehrere, vorzugsweise zwei, einander benachbart angeordnete Auflageelemente. Die Auflageelemente sind vorzugsweise derart angeordnet, daß diese in Applikationsposition einer Atemmaskeneinrichtung oberhalb der linken und rechten Augenbrauen des Maskenanwenders positioniert sind. Die beiden Auflageelemente sind vorzugsweise über eine flexible Stegeinrichtung miteinander verbunden. Hierdurch kann zum einen eine weitere Vergrößerung der Auflagefläche erreicht und zudem die Verdrehmöglichkeit der Auflageelemente zueinander definiert eingeschränkt werden. Insbesondere bei dieser Ausführungsform sind die beiden Auflageelemente vorzugsweise miteinander integral ausgebildet. Die Gestalt der Auflageelemente in einer Draufsicht ist nicht auf im wesentlichen kreisförmige Außenkonturen beschränkt. Beispielsweise ist es auch möglich, elliptische oder andere polygonale Außenkonturen zu wählen.

Die Gelenkeinrichtung zur schwenkbewegbaren Lagerung des jeweiligen Auflageelementes ist vorzugsweise ebenfalls integral mit dem Auflageelement ausgebildet. Ein definierter Gelenkcharakter kann durch entsprechende Geometriegestaltung der Gelenkeinrichtung erreicht werden.

Die Gelenkeinrichtung ist vorzugsweise derart angeordnet, daß sich diese auf oder zumindest nahe einer Kraftwirkungslinie befindet, die durch einen Flächenpressungsschwerpunkt des jeweiligen Auflageelementes verläuft. Hierdurch wird die Vergleichmäßigung der Flächenpressungsverteilung noch weiter unterstützt.

Die Auflageelemente sind vorzugsweise derart profiliert ausgebildet, daß ein Ansaugen des Auflageelementes auf der Stirnfläche des Patienten verhindert wird. Einem Ansaugen der Stirnauflageeinrichtung auf der Stirnfläche des Patienten kann weiterhin auch dadurch vorgebeugt werden, daß das Auflageelement mit Durchgangsbohrungen oder auch mit Kanälen versehen ist, durch welche Luft in einen Zwischenbereich zwischen dem Auflageelement und der Stirn des Maskenanwenders eintreten kann.

Durch die Kugelgelenkstrukturen wird in vorteilhafter Weise eine gute Anpaßbarkeit an den horizontalen sowie an der vertikalen Stirnverlauf des Maskenanwenders erreicht. Die Gelenkeinrichtung - insbesondere die Kugelgelenkeinrichtung - kann auch feststellbar ausgerichtet sein. Die Gelenkeinrichtung kann in weiterhin vorteilhafter Weise auch um bestimmt Achsen - insbesondere um eine Horizontalachse - bevorzugt kippbar sein. Die Wölbung des Auflageelementes kann derart gewählt sein, daß sich in Horizontal- und Vertikalrichtung unterschiedliche Krümmungsradien ergeben. Die Krümmungsradien sind vorzugsweise kleiner gewählt, als übliche Stirnkrümmungsradien.

Alternativ zu Kugelgelenkstrukturen sind auch kardanische Aufhängungen - beispielsweise mittels Gelenkstift möglich. Der Schwenkwinkel der Gelenkeinrichtung ist vorzugsweise auf einen vorgegebenen Anschlagwinkel begrenzt. Die Materialeigenschaften des Auflageelementes sind vorzugsweise derart gewählt, daß sich dieses im wesentlichen antibakteriell und ggf. wundheilungsfördernd verhält.

In vorteilhafter Weise kann im Bereich der Auflagefläche eine Polstereinrichtung, insbesondere eine Gelkörperpolstereinrichtung, oder auch eine Luft- oder Flüssigkeitspolstereinrichtung ausgebildet sein. Durch Variation der eingebrachten Flüssigkeit-, Luft- oder Gelmenge kann hierbei auf vorteilhafte Weise die Position der Atemmaske relativ zum Maskenanwender eingestellt werden.

In vorteilhafter Weise ist die Lagerungsposition der Auflageelemente in einstellbarer Weise veränderbar. Alternativ hierzu - oder auch in Kombination mit dieser Maßnahme ist es auch möglich, mehrere Kopplungsmöglichkeiten vorzusehen, so daß sich verschiedene Stirnabstände durch entsprechende, selektive Koppelung erreichen lassen. Es ist möglich, durch beispielsweise 2, vorzugsweise 3 oder auch mehrere Kopplungsmöglichkeiten eine GrobEinstellung vorzunehmen und innerhalb eines begrenzten Feinjustierbereich eine vorzugsweise stufenlose Feinjustierung vorzunehmen. Es ist möglich mehrere Permutationsmöglichkeiten zu erlauben, wobei die einzelnen Koppelungspermutationen zu jeweils unterschiedlichen Einstellungen bezüglich des Stirnabstandes führen. Es ist auch möglich, Klemmstrukturen vorzusehen durch welche eine stufenlose Einstellung des Stirnabstandes möglich ist. Die Koppelungsstellen können derart ausgebildet sein, daß eine definierte Klebestelle erreicht wird, so daß eine stufenlos individuell angepaßte Einstellung durch Klebung dauerhaft erhalten bleibt.

Ein besonders hoher Tragekomfort wird gemäß einer besonders bevorzugten Ausführungsform der Erfindung dadurch erreicht, daß die mit der Hautfläche des Maskenanwenders in Kontakt gelangenden Oberflächenabschnitte des Auflageelementes eine samtartig matte Oberfläche aufweisen. Gem. einer besonders bevorzugten Ausführungsform der Erfindung ist zumindest im Bereich der Auflagefläche des Auflageelementes eine Oberflächenstruktur zur Erreichung eines Selbstreinigungseffektes vorgesehen. Eine derartige Oberflächenstruktur kann beispielsweise Lotusblatt-Oberflächenstrukturen aufweisen. Das Auflageelement kann auch, zumindest im Bereich der Auflagefläche, mit einem Gelkörper versehen sein.

Eine noch weiter erhöhte Anpassbarkeit der Stirnauflageeinrichtung an die individuelle Krümmung der Stirn des Maskenanwenders, wird gemäß einem weiteren Aspekt der vorliegenden Erfindung dadurch erreicht, dass die Schwenkachsen der Armteile zueinander um einen Winkel α im Bereich von 8 bis 45° zueinander geneigt sind. Es ist in vorteilhafter Weise möglich, die, die Schwenkachsen festlegende Struktur derart auszubilden, dass der Winkel α der Schwenkachsen zueinander, in einstellbarer Weise veränderbar ist.

Vorzugsweise sind die Schwenkachsen derart festgelegt, dass sich diese bezogen auf eine Frontalansicht eines Maskenanwenders im Bereich zwischen der die Augenbrauen verbindenden Querlinie und dem Kinn des Maskenanwenders schneiden. Hierbei wird eine besonders hohe Kompatibilität zu den statistisch überwiegend vorherrschenden Gesichtstekturen erreicht.

Die Schwenkachsen sind vorzugsweise jeweils durch eine Scharniereinrichtung definiert. Diese Scharniereinrichtungen können als mehrteilige Gelenkvornchtungen aufgebaut sein, oder gemäß einer besonders bevorzugten Ausführungsform der Erfindung als Filmscharniere ausgebildet sein. Bei dieser Ausführungsform ist es möglich, die Armteile sowie die zur Anlenkung der Armteile vorgesehene Gelenkbasisstruktur, einstückig d.h. integral aus einem Kunststoffmaterial zu fertigen.

### Kurzbeschreibung der Zeichnungen

Weitere Einzelheiten und Merkmale der Erfindung ergeben sich aus der nachfolgenden Beschreibung in Verbindung mit der Zeichnung. Es zeigt:
- **Fig. 1**: eine perspektivische Ansicht einer erfindungsgemäßen Atemmaskenanordnung;
- **Fig. 2**: eine weitere perspektivische Ansicht der erfindungsgemäßen Atemmaskenanord- nung ohne Schlauchanschlusshülse;
- **Fig. 3**: eine perspektivische Ansicht der Applikationsstruktur der vorangehend gezeigten Atemmaskenanodnung;
- **Fig. 4**: eine perspektivische Ansicht der Applikationsstruktur mit daran angeschlossenem Dockingport;
- **Fig. 5**: eine perspektivische Ansicht eines, aus Trägerabschnitt und Rahmenabschnitt gebildeten Integralteils;
- **Fig. 6**: eine weitere perspektivische Darstellung des Integralteils nach Fig. 5 zur Erläute- rung der zur Ankoppelung des Dockingports vorgesehenen Verrastungseinrich- tung;
- **Fig. 7**: eine Seitenansicht der erfindungsgemäßen Atemmaskenanordnung;
- **Fig. 8**: eine vereinfachte perspektivische Ansicht einer Stirnauflageeinrichtung mit zwei schwenkbewegbar gelagerten padartigen Auflageelementen sowie andeutungs- weise einer Atemmaske;
- **Fig. 9**: eine vereinfachte Schnittansicht zur Erläuterung einer bevorzugten Ausführungs- form eines schwenkbewegbar gelagerten Auflageelementes;
- **Fig. 10**: eine Draufsicht auf zwei integral miteinander ausgebildete Auflageelemente, von welchen jedes individuell mit einer Schwenklagerungseinrichtung versehen ist;
- **Fig. 11**: eine weitere Ausführungsform eines Auflageelementes mit einer konkav gewölb- ten Auflagefläche und einem Kalottenabschnitt zur Lagerung des Auflageelemen- tes in schwenkbewegbarer Weise;
- **Fig. 12**: eine Skizze zur Erläuterung einer bevorzugten Flächenpressungsverteilung von einem Mittenbereich des Auflageelementes in ihrem Verlauf von einem Zent- rumsbereich des Auflageelementes zu dessen Randbereich hin.
- **Fig. 13**: eine Frontalansicht des Gesichtsbereichs einer Person zur Erläuterung bevorzugter Auflagezonen der Stirnauflageeinrichtung sowie bevorzugte Ausrichtungen der für die auslenkbare Lagerung der Armelemente maßgeblichen Schwenkachsen;
- **Fig. 14**: eine perspektivische Darstellung zur Erläuterung einer Stelltriebeinrichtung zum Schwenken der Armelemente in einstellbarer Weise;
- **Fig. 15**: eine Detailschnittansicht zur Erläuterung des Aufbaus einer mit Filmscharnier- strukturen versehen Variante des Stelltriebs;
- **Fig. 16**: eine weitere Detailschnittdarstellung zur Erläuterung einer Ausführungsform der Stelltriebeinrichtung ebenfalls mit einem Filmscharnier und einem, mit einem unmittelbar mit einem Eingriffsabschnitt eines Armelementes in Eingriff stehen- den Spiralnutbereich der an einer Unterseite des Stellrades ausgebildet ist;
- **Fig. 17**: eine vereinfachte Schnittansicht einer weiteren Ausführungsform einer Stelltrieb- einrichtung mit einem, über eine Drehzapfenzone schwenkbar angelenkten Armelement;
- **Fig. 18**: eine vereinfachte Seitenansicht einer, mit einer Atemmaskenanordnung versehe- nen Applikationseinrichtung;
- **Fig. 19**: eine vereinfachte perspektivische Ansicht einer Applikationseinrichtung mit integrierter Stirnauflageeinrichtung zur Applikation einer Atemmaske;
- **Fig. 20**: eine Seitenansicht einer Atemmasken-Applikationseinrichtung mit einer darin schwenkbewegbar aufgenommenen Atemmaskenanordnung;
- **Fig.21**: eine Detailansicht eines zur Koppelung mit einer Atemmaskenanordnung vorgesehenen Halteabschnittes einer Applikationseinrichtung;
- **Fig. 22**: eine perspektivische Ansicht einer Applikationseinrichtung für eine Atemmasken- anordnung wie sie beispielsweise auf einen Atemgas-Zuleitungsabschnitt einer Atemmaske aufsetzbar ist.
- **Fig. 23**: eine perspektivische Ansicht einer Stirnabstützeinrichtung mit einem Kopfband- abschnitt und einer damit verbundenen Koppelungsstruktur zum Abstützen eines Leitungsabschnittes;
- **Fig. 24**: eine perspektivische Ansicht einer weiteren Ausführungsform einer Stirnabstütz- einrichtung mit einem Einstellmechanismus zum Einstellen des Stirnabstandes sowie der seitlichen Position eines Leitungsabschnittes;
- **Fig. 26**: eine Detail-Ansicht eines Endbereiches eines Stirnauflageabschnittes mit flach auslaufender Bügelunterlage;
- **Fig. 27**: eine Teilezkizze zur Erläuterung einer Schraubenspindelvariante eines Stelltrieben wie er beispielsweise bei der Ausführungsformnachden Figuren 24 und 25 An- wendung finden kann;
- **Fig. 28**: eine Skizze zur Erläuterung eines aus einem Elastomer-Material gefertigten Leitungsabschnittes mit integralem, in einer Stirnabstützeinrichtung abstützbaren Masken-Anschlusssockel;
- **Fig. 29**: eine Schnittansicht zur Erläuterung einer durch Poren oder Kavernen gepolstert ausgebildeten Variante des Stirnabstützabschnittes;
- **Fig. 30**: eine Schnittansicht durch einen im Bereich der Stirnabstützung gebildten Verbin- dungsbereich zwischen dem Anschlussstutzen einer Atemmaske und einem Lei- tungsabschnitt (hier maskenseitige Buchse eines Auswaschadapters - AERO-CLICK (eingetragenen Marke der Firma MAP Medizin-Technologie GmbH); Wisper-Swivel (Respironics));
- **Fig.31**: eine Skizze zur Erläuterung einer weiteren erfindungsgemäßen Variante einer Stirnabstützeinrichtung;
- **Fig. 32**: eine Ausführungsform eines Anschlussadapters mit daran einstellbar angelenkter Stirnabstützung;
- **Fig. 33**: eine Draht-Biegevariante der Stirnabstützung aus Fig. 32;
- **Fig. 34**: eine Gewinde Buchsen/ Spindelvariante der Einstelleinrichtung aus Fig. 32;
- **Fig. 35**: eine Ausführungsform einer Atemmaskenanordnung mit stufenlos krümmungs- veränderbarem Rohrleitungsabschnitt - und damit gekoppelter Simabstützeinrich- tung;
- **Fig. 36**: eine Seitenansicht einer weiteren Variante eines Krümmungsveränderbaren Leitungsabschnittes;
- **Fig. 37**: eine perspektivische Ansicht einer Kopfbandanordnung zur insbesondere zur Verwendung mit den vorangehend beschriebenen Stirnabstützeinrichtungen.

### Ausführliche Beschreibung der Zeichnungen

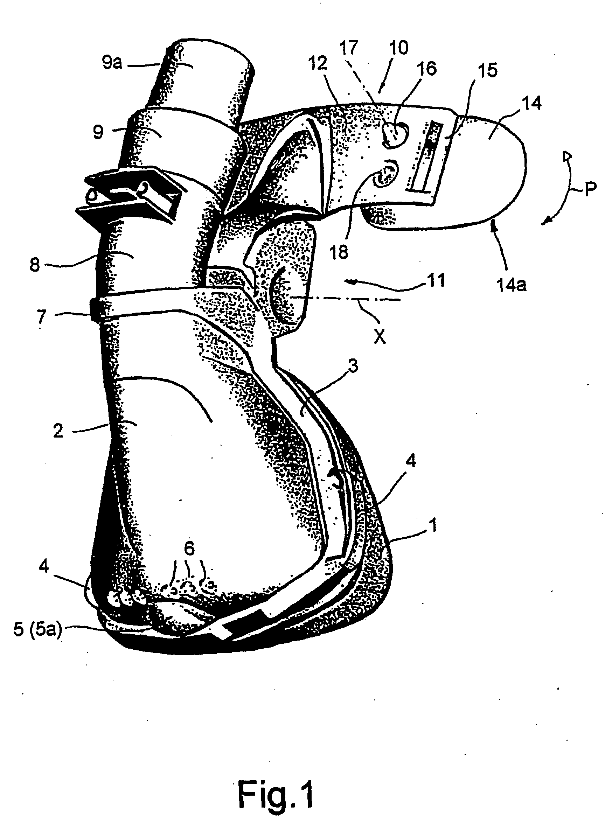

Die in Fig. 1 dargestellte Atemmaskenanordnung umfasst eine, aus einem Elastomermaterial, insbesondere Silikonkautschuk gefertigte Dichtlippeneinrichtung 1 und einen Gewölbekörper 2. Die Dichtlippeneinrichtung 1 ist derart ausgebildet, dass diese eine, zur Aufnahme des Nasenbereichs eines Maskenanwenders vorgesehene Aufnahmeöffnung umsäumt und hierbei vorzugsweise den Nasenrücken sowie den Oberlippenbereich des Maskenanwenders überquert. Die Dichtlippeneinrichtung 1 hat hierbei eine im wesentlichen sattelförmige Silhouette. Die Dichtlippeneinrichtung 1 und der Gewölbekörper 2 sind bei diesem Ausführungsbeispiel einstückig aus einem Elastomermaterial ausgebildet und in einem Rahmenabschnitt 3 aufgenommen.

Der Rahmenabschnitt 3 ist aus einem Kunststoffmaterial gefertigt und weist integral mit diesem, gefertigte Haltebügel 4 auf. Die Haltebügel 4 befinden sich in Applikationsposition der Atemmaskenanordnung auf Wangenhöhe oder auf Höhe der Nasenflügel des Maskenanwenders und ermöglichen die Ankoppelung einer unteren Gurtbandanordnung. Zur zuverlässigen Koppelung des Gewölbekörpers 2 mit dem Rahmenabschnitt 3 ist eine Raststruktur 5 vorgesehen, über welche der Gewölbekörper 2 an dem Rahmenabschnitt 3 in Fügeposition durch Einrasten fixierbar ist. Die Raststruktur umfasst eine, eine Oberseite des Rahmenabschnitts 3 übergreifende Rastnase 5a.

An dem Gewölbekörper 2 sind mehrere Auslassöffnungen 6 ausgebildet, zur Ableitung verbrauchten Atemgases an die Umgebung.

Die Atemmaskenanordnung umfasst weiterhin einen Trägerabschnitt 7, der bei diesem Ausführungsbeispiel integral mit dem Rahmenabschnitt 3 ausgebildet ist.

An dem Trägerabschnitt 7 ist ein hier als Dockingport 8 ausgebildetes Atemgasleitungsorgan lösbar fixiert. Der Dockingport 8 umfasst einen, hier nicht sichtbaren Ringflansch, auf welchen eine Schlauchanschlusshülse 9 drehbewegbar aufgesetzt ist. Die Schlauchanschlusshülse 9 umfasst einen Schlauchanschlussstutzenabschnitt 9a auf den ein Endabschnitt eines Atemgasschlauches aufsteckbar ist.

Die erfindungsgemäße Atemmaskenanordnung umfasst weiterhin eine Stirnabstützeinrichtung 10, die über eine Einstelleinrichtung 11 bewegbar mit dem Rahmenabschnitt 3 gekoppelt ist.

Die Einstelleinrichtung 11 ist derart ausgebildet, dass diese ein Verschwenken der Stirnabstützeinrichtung 10 gegenüber dem Rahmenabschnitt 3 um die hier dargestellte Schwenkachse X ermöglicht. Die Einstelleinrichtung 11 umfasst einen, Fixiermechanismus, durch welchen die Stirnabstützeinrichtung 10 und der Rahmenabschnitt 3 in der gewählten Relativposition fixiert werden können.

Die Stirnabstützeinrichtung 10 umfasst einen Bügelabschnitt 12 an welchem Stirnpolsterelemente 14 anbringbar sind. An dem Bügelabschnitt 12, sind ähnlich wie an dem Rahmenabschnitt 3, Haltebügel 15 ausgebildet, zur Koppelung des Bügelabschnitts 12 mit einer oberen Gurtbandanordnung eines zur Fixierung der Atemmaskenanordnung vorgesehenen Kopfbandes.

Die Stirnpolsterelemente 14 sind aus einem Elastomermaterial gefertigt und bilden auf ihrer, in Applikationsposition dem Maskenanwender zugewandten Unterseite 14a eine Auflagefläche mit einer vorgegebenen Flächenpressungsverteilung. Die Stirnpolsterelemente 14 sind jeweils über einen Steckfuss 16 mit dem Bügelabschnitt 12 gekoppelt. Der Steckfuss 16 ist derart exzentrisch an dem jeweiligen Stirnpolsterelement 14 ausgebildet, dass durch Schwenken der Stirnpolsterelemente 14 wie durch den Pfeil P angedeutet, um die Steckfussachse 17 unterschiedliche Auflagepositionen der Unterseite 14a der Stirnpolsterelemente 14 auf der Stirnfläche des Maskenanwenders erreicht werden können. Durch Auswahl der Schwenkposition des Stirnpolsterelementes 14 sowie durch Auswahl der zur Aufnahme des Steckfusses 16 vorgesehenen Aufnahmeöffnung 18 können unterschiedliche Auflagepositionen erreicht werden. Bei dem hier gezeigten Ausführungsbeispiel sind für das linke und das rechte Stirnpolsterelement 14 jeweils 2 voneinander beabstandete Aufnahmeöffnungen 18 in dem Bügelabschnitt 12 ausgebildet.

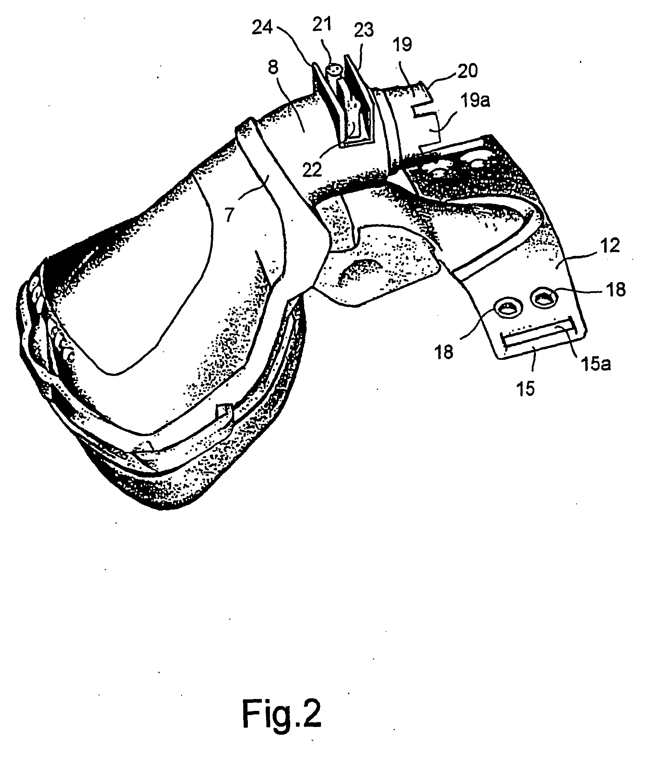

Die Stirnpolsterelemente 14 können durch Herausziehen der Steckfüsse 16 aus den Aufnahmeöffnungen 18 von dem Bügelabschnitt 12 abgenommen werden, wie dies in Fig. 2 gezeigt ist. Die Mittelachsen der Aufnahmeöffnungen 18 sind bei diesem Ausführungsbeispiel um etwa 10 mm voneinander beabstandet. Die Exzentrizität des an dem jeweiligen Stirnpolsterelement 14 ausgebildeten Steckfusses 16 (s. hierzu Fig. 1) beträgt ebenfalls ca. 10 mm. Infolge der exzentrischen Anordnung des Steckfusses 16 sowie der beabstandeten Ausbildung der Aufnahmeöffnung 18, wird im wesentlichen senkrecht zu einer die Augenbrauen des Maskenanwenders verbindenden Linie, eine Höhenveränderbarkeit der Stirnpolsterelemente im Bereich von ca. 30 mm erreicht. In seitlicher Richtung, d.h. in Richtung der genannten, die Augenbrauen verbindenden Linie, ist ebenfalls eine Veränderung der Auflageposition im Bereich von 20 mm möglich. Der zur Ankoppelung einer oberen Gurtbandanordnung vorgesehene Haltebügel 15 ist derart nahe an den Aufnahmeöffnungen 18 angeordnet, dass die durch den Haltebügel begrenzte Durchführungsöffnung 15a zum Maskenanwender hin, durch das Stirnpolsterelement 14 abgedeckt ist.

In der Darstellung gemäß Fig. 2 ist die in Fig. 1 gezeigte Schlauchanschlusshülse 9 von dem Dockingport 8 abgenommen. Erkennbar ist in dieser Ansicht ein integral mit dem Dockingport 8 ausgebildeter Ringflansch 19, der mehrere Zungenelemente 19a aufweist, die zum Zentrum des durch den Dockingport 8 gebildeten Durchgangskanals hin elastisch auslenkbar sind. An den Zungenelementen 19a ist ein Rastwulst 20 ausgebildet der mit einer komplementär an der Schlauchanschlusshülse 9 ausgebildeten Innenumfangsnut, in Eingriff bringbar ist. Die Geometrie des Rastwulstes 20, der in der Drehhülse 9 ausgebildeten Innenumfangsnut sowie die Elastizität der Zungenelemente 19a ist derart abgestimmt, dass die Schlauchanschlusshülse 9 bei Überschreiten einer vorbestimmten Zugkraft werkzeuglos von dem Ringflansch 19 abziehbar ist. Der Ringflansch 19 und die Schlauchanschlusshülse 9 sind weiterhin derart passend ausgebildet, dass die Schlauchanschlusshülse 9 leicht drehbar auf dem Ringflansch 19 sitzt.

Der Dockingport 8 ist bei diesem Ausführungsbeispiel mit Schlauchanschlussstutzen 21, 22 versehen auf die ein Stecker oder Schlauchelement aufsteckbar ist. Die Schlauchanschlussstutzen 21, 22 bilden jeweils einen Durchgangskanal der in den in dem Dockingport 8 gebildeten Atemgaskanal mündet. Die Schlauchanschlussstutzen 21, 22, sind bei Nichtgebrauch durch ein Pfropfen- oder Kappenelement (nicht dargestellt) verschliessbar, das vorzugsweise reibschlüssig auf diesen Schlauchanschlussstutzen 21, 22 sitzt. Bei dieser Ausführungsform sind die Schlauchanschlussstutzen 21, 22 in einer Nut versenkt angeordnet, die durch zwei aufragende Nutwände 23, 24 begrenzt ist. Der Dockingport 8 ist an dem Trägerabschnitt 7 über eine Steckverbindungsstruktur gekoppelt.

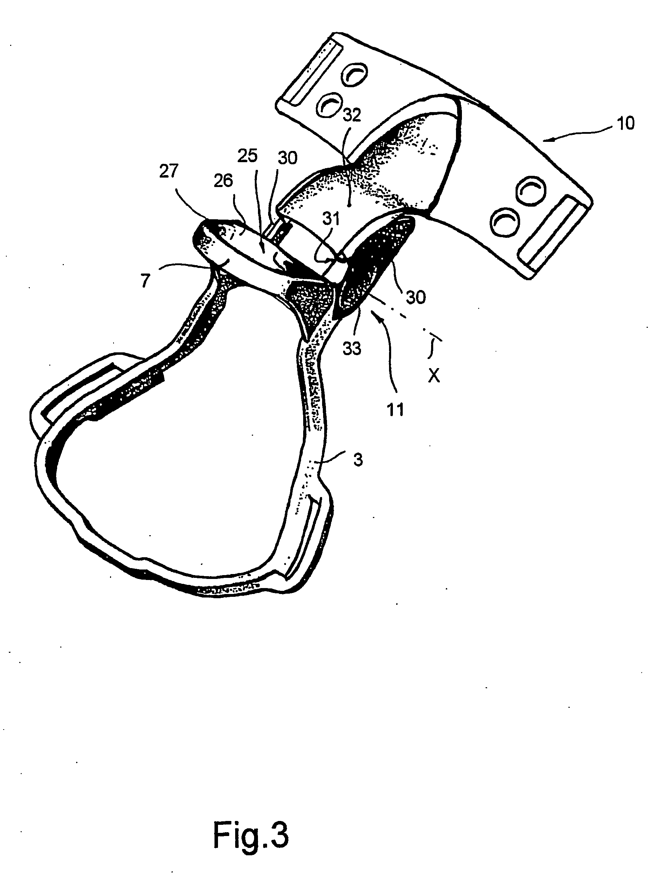

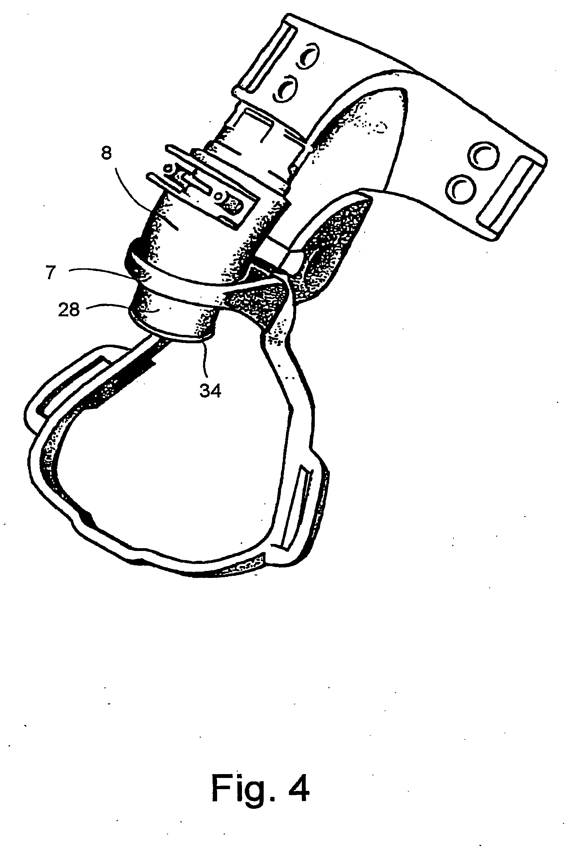

Die Rastverbindungsstruktur zur Koppelung des Dockingports 8 mit dem Trägerabschnitt 7, ist wie aus der Darstellung nach Fig. 3 ersichtlich, bei diesem Ausführungsbeispiel als Drehverrastungs- bzw. als Bajonettverbindungsstruktur 25 ausgebildet. Die Bajonettverbindungsstruktur 25 umfasst zwei einander diametral gegenüberliegende und durch Einsatzfalze 26 voneinander getrennte Rastbrücken 27. Die Rastbrücken 27 geraten in Verriegelungsposition mit zwei Rastvorsprüngen in Eingriff, die an einem Einsteckflanschabschnitt 28 (Fig. 4) des Dockingports ausgebildet sind.

Der Trägerabschnitt 7 ist integral mit dem Rahmenabschnitt 3 ausgebildet und bildet dabei die zur Aufnahme des Einsatzstutzens 28 vorgesehene und partiell von den Rastbrücken 27 umsäumte Einsatzöffnung. Im Übergangsbereich zwischen dem Trägerabschnitt 7 und dem Rahmenabschnitt 3 sind zwei Führungsflanken 30 vorgesehen, die ebenfalls integral mit dem Rahmenabschnitt 3 ausgebildet sind und Teil der Einstelleinrichtung 11 bilden. Die Führungsflanken 30 bilden eine Bogenführung 31 in welcher ein Koppelungsabschnitt 32 der Stirnabstützeinrichtung 10 verschiebbar geführt ist Die Bogenführung 31 sowie der darin geführten Bereich des Koppelungsabschnitts 32 sind derart ausgebildet, dass die Stirnabstützeinrichtung 10 und der Rahmenabschnitt 3 zueinander um die bereits in Fig. 1 gezeigte Schwenkachse X bewegbar sind. An den Führungsflanken 30 ist eine Betätigungszone 33 ausgebildet, zur Aufbringung einer die Einstelleinrichtung 11 in eine Freigabestellung bringenden Entriegelungskraft. Die Entriegelungskraft kann insbesondere bei Umgreifen des Dockingports 8, mit dem Daumen und dem Zeigefinger und Auflage der entsprechenden Fingerspitzen, auf der Betätigungszone 33 aufgebracht werden. Alternativ zu der hier vorgesehenen Bogenführung 31, ist es auch möglich, die Einstelleinrichtung 11 derart auszubilden, dass eine Relativbewegung zwischen der Stirnabstützeinrichtung 10 und dem Rahmenabschnitt 3 entlang einer von einer Bogenbahn abweichenden Bewegungsbahn ermöglicht ist. An der Stirnabstützeinrichtung 10 können auch Stirnpolstereinrichtungen angebracht werden, die in ihrem Aufbau von den Stimpolsterelementen 14 gemäß Fig. 1 abweichen.

In Fig. 4 ist die Applikationsstruktur der erfindungsgemäßen Atemmaskenanordnung in einem Montagezustand dargestellt, bei welchem der erfindungsgemäße Dockingport 8 in die, in dem Trägerabschnitt 7 gebildete Einsatzöffnung eingesetzt - und über die, im Bereich der Einsatzöffnung ausgebildete Bajonettverschlusseinrichtung lagerichtig fixiert ist. Der Dockingport 8 bildet bei dieser Ausführungsform ein rohrkrümmerartiges Leitungsorgan, durch welches das über die Schlauchanschlusshülse 9 (Fig. 1) zufliessende Atemgas um einen Winkel von etwa 30° zur Nasenspitze des Maskenanwenders hin abgelenkt wird. Durch die derartige Zuführung des Atemgases zu dem von dem Gewölbekörper 2 (Fig. 1) begrenzten Maskeninnenraum wird eine vorteilhafte Überströmung des Nasenrückens des Maskenanwenders erreicht.

Durch den modularen Aufbau der erfindungsgemäßen Atemmaskenanordnung wird es möglich, eine dem jeweiligen Anwendungsfall verbessert Rechnung tragende Konfiguration der Atemmaskenanordnung vorzunehmen. Der in den Trägerabschnitt 7 eingesetzte Dockingport 8 weist einen Einsatzstutzen 28 auf, der über den Trägerabschnitt 7 zu dem Rahmenabschnitt 3 hin hervorkragt. Auf diesen Einsatzstutzen 28 ist unter geringer elastischer Aufweitung ein Einsatzöffnungsabschnitt des Gewölbekörpers 2 aufsetzbar. An dem Einsatzstutzen 28 ist ein Umfangswulst 34 ausgebildet, durch welchen der Gewölbekörper 2 und der Einsatzstutzen 28 in einer definierten Fügestellung gehalten werden. Dieser Umfangswulst 34 kann mit einer entsprechend in dem Gewölbekörper 2 ausgebildeten Innenumfangsnut in Eingriff treten oder auch auf einer Innenfläche des Gewölbekörpers 2 aufsitzen. Der Trägerabschnitt 7 und der Dockingport 8 sind derart ausgebildet, dass der Dockingport einen, den Nasenwurzelbereich überbrückenden Atemgasleitungsabschnitt bildet. Hierdurch wird eine geringe Beeinträchtigung des Sichtfeldes erreicht.

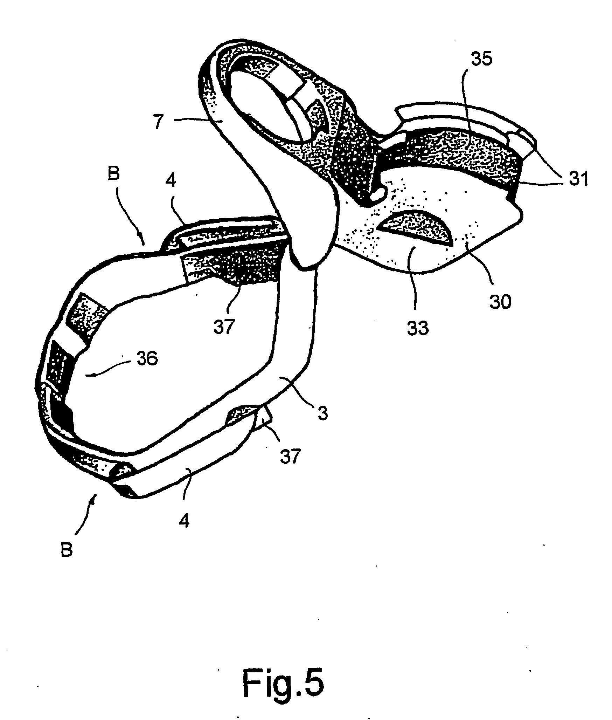

In Fig. 5 ist eine weitere perspektivische Ansicht des den Rahmenabschnitt 3 bildenden Integralteils dargestellt. Die einstückig mit dem Rahmenabschnitt 3 gefertigten Führungsflanken 30, sind derart ausgebildet, dass die Aufbringung einer Lösekraft auf die Betätigungszonen 33 die Führungsflanken 30 im Bereich der Bogenführung 31 auseinanderdrängt. Beim vorliegenden Ausführungsbeispiel wird hierzu durch einen Bogensteg 35 ein Hebel/Kippeffekt erzielt. Die Rückstellung der Führungsflanken 30 im Bereich der Bogenführung 31 erfolgt in Folge der Materialeigenelastizität. Die Fixierwirkung kann im Wege einer reibschlüssigen Klemmung der zwischen den Führungsflanken 30 geführten Kontaktzonen des Koppelungsabschnitts 32 erfolgen. Alternativ hierzu oder in Kombination damit, ist es auch möglich, die Fixierwirkung durch formschlüssige Koppelung, beispielsweise im Wege einer Fein-Verzahnung zu erreichen.

Vorzugsweise ist eine Anschlageinrichtung vorgesehen, die den maximalen Schwenkbereich der Stirnabstützeinrichtung 10 gegenüber dem Rahmenabschnitt 3 begrenzt. Es ist möglich, diese Anschlageinrichtung derart auszubilden, dass diese beispielsweise durch Vergrößern der auf die Betätigungszone 33 aufgebrachten Betätigungskräfte in einen Zustand gelangt in welchem die Stirnabstützeinrichtung 10, von der mit dem Rahmenabschnitt 3 verbundenen Gelenkstruktur, trennbar ist.

In der Darstellung nach Fig. 5 ist weiterhin ein Ausbuchtungsbereich 36 des Rahmenabschnitts 3 erkennbar, durch welchen die Positionierung und Fixierung des Gewölbekörpers 2 in dem Rahmenabschnitt 3 verbessert ist. Die Positionierung des Gewölbekörpers 2 in dem Rahmenabschnitt 3 wird weiterhin unterstützt, durch zwei Fixierschilde 37 die im Bereich der Haltebügel 4 an der Unterseite des Rahmenabschnitts 3 ausgebildet sind und in Klemmtaschenabschnitte eintauchen, die im Übergangsbereich der Dichtlippeneinrichtung 1 in dem Gewölbekörper 2 ausgebildet sind. Der Rahmenabschnitt 3 weist eine im wesentlichen sattelförmige Silhouette auf und erreicht seine maximale Breite in einer Zone des Rahmenabschnittes 3, die sich in Applikationsposition der Atemmaskenanordnung in etwa auf Höhe der Nasenflügel des Maskenanwenders befindet. Die Haltebügel 4 erstrecken sich von dieser Zone B maximaler Breite zum Augenbereich des Maskenanwenders hin, aufwärts. Die Innenkanten der von den Haltebügeln 4 umsäumten Durchführungsöffnungen sind gerundet ausgebildet, um ein etwaiges Durchscheuern der durch diese Öffnungen hindurchgeführten Bandabschnitte zu vermeiden. In dieser Darstellung ist die zur Fixierung des Dockingports in dem Trägerabschnitt 7 ausgebildete Verrastungseinrichtung nochmals erkennbar.

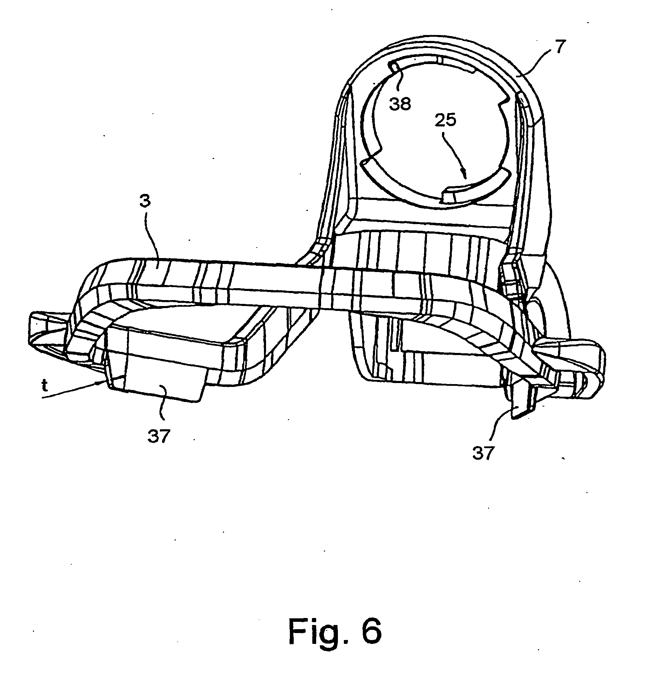

In Fig. 6 sind die an der Unterseite des Rahmenabschnitts 3 ausgebildeten Fixierschilde 37 aus einer anderen Perspektive erkennbar. Die Fixierschilde 37 weisen eine, quer zur Fügerichtung gemessene Dicke t im Bereich von 0,8 bis 3 mm auf. Die Fixierschilde sind in Fügerichtung verjüngt ausgebildet. Die Fixierschilde 37 sowie insbesondere auch der an dem Gewölbekörper 2 anliegende Innenumfangsbereich des Rahmenabschnitts 3 kann mit einer die Koppelung des Gewölbekörpers noch weiter unterstützenden Profilierung versehen sein. Vorzugsweise sind feine, in Umfangsrichtung des Rahmenabschnitts 3 verlaufende Profilrillen an dem Rahmenabschnitt sowie an dem hieran anliegenden Abschnitt des Gewölbekörpers 2 ausgebildet.

Die an dem Trägerabschnitt 7 ausgebildete Bajonettverbindungsstruktur 25 umfasst einen Endanschlag 38, durch welchen die Endposition des Dockingports 8 in Koppelungsstellung festgelegt ist. Die Bajonettverbindungsstruktur 25 ist bei diesem Ausführungsbeispiel derart ausgebildet, dass die maximale Fixierkraft in der durch den Endanschlag 38 festgelegten Endstellung erreicht wird.

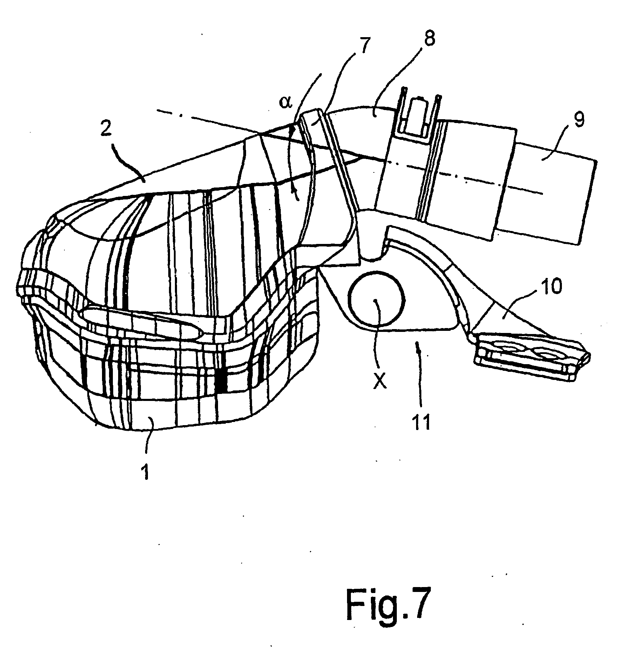

In Fig. 7 ist die erfindungsgemäße Atemmaskenanordnung - mit Ausnahme der Stirnpolsterelemente 14 ( Fig.1) - in vollständig zusammengebauten Zustand aus Richtung der Schwenkachse X gesehen, dargestellt. Wie in dieser Ansicht erkennbar, bildet der erfindungsgemäß lösbar in den Trägerabschnitt 7 einsetzbare Dockingport 8, ein Koppelungselement zur Koppelung des elastomeren Gewölbekörpers 2, mit einer drehbar gelagerten Schlauchanschlusshülse 9, durch welches das zuströmende Atemgas um einen Winkel α abgelenkt wird, der bei diesem Ausführungsbeispiel 32° beträgt.

Der Dockingport 8 kann aus einem mehrere Dockingports 8 enthaltenen Satz ausgewählt sein und bildet ein Schnittstellenteil, durch welches der Gewölbekörper 2 mit unterschiedlichen Schlauchsystemen koppelbar ist. Auch über die auf den Dockingport 8 aufgesetzte Schlauchanschlusshülse 9 kann die Kompatibilität zu unterschiedlichen Atemgasleitungssystemen sichergestellt werden. Es ist möglich, mehrere, jeweils auf bestimmte Gesichtstypen abgestimmte Dichtlippeneinrichtungen vorzusehen und eine bedarfsgerechte Konfiguration der erfindungsgemäßen Atemmaskenanordnung dadurch zu erreichen, dass ein, der individuellen Gesichtstektur des Maskenanwenders besonders gut Rechnung tragendes, aus Dichtlippeneinrichtung 1 und Gewölbekörper 2 bestehendes Elastomerelement, in die erfindungsgemäße Maskenanordnung integriert wird. Es ist auch möglich, mehrere, jedoch zur Einstelleinrichtung 11 kompatible Varianten der Stirnauflageeinrichtung vorzusehen und die erfindungsgemäße Maskenanordnung durch eine, der individuellen Gesichtstektur besonders gut Rechnung tragende Variante der Stirnabstützeinrichtung 10 zu bestücken. Die Stirnabstützeinrichtung 10 kann alternativ zu den in Fig. 1 dargestellten Stirnpolsterelementen auch mit anderweitigen Stirnpolstereinrichtungen zur gepolsterten Auflage auf der Stirn des Maskenanwenders bestückt werden. Es ist möglich, derartige Polstereinrichtungen, beispielsweise in eine obere Stirnbandanordnung zu integrieren und die Stirnabstützeinrichtung, beispielsweise über eine Klettverbindungsstruktur, auf diese gepolsterte Stirnbandanordnung aufzusetzen.

Die Erfindung ist nicht auf das vorangehende Ausführungsbeispiel beschränkt. Beispielsweise ist es auch möglich, in den erfindungsgemäß ausgestalteten Rahmenabschnitt 3 einen Gewölbekörper 2 einzusetzen der nicht aus einem Elastomermaterial gefertigt ist.

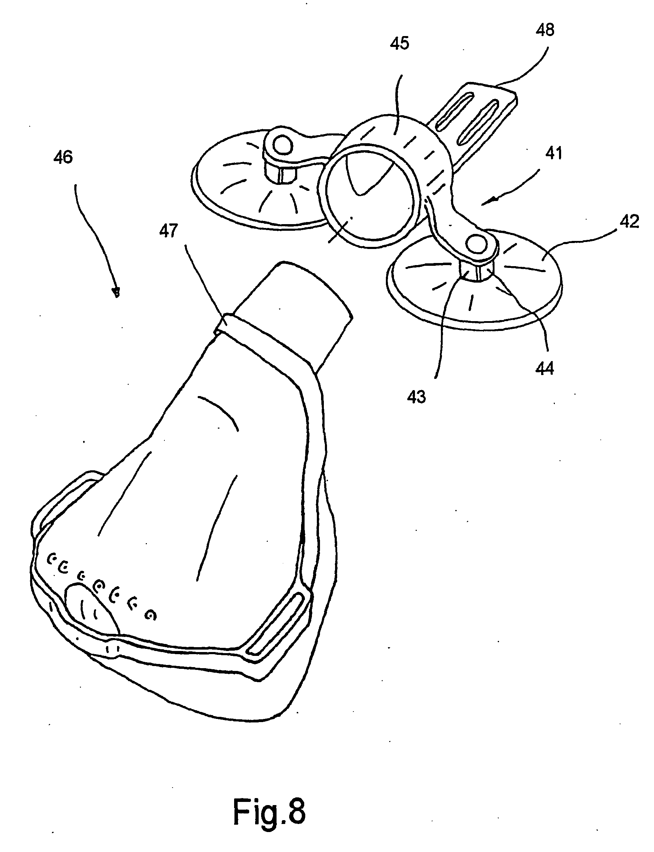

Die in Fig. 8 gezeigte Stirnauflageeinrichtung umfaßt eine Halteeinrichtung 41, zur Halterung eines Auflageelementes 42 in schwenkbewegbarer Weise. Die Halteeinrichtung 41 umfaßt hierzu eine Schwenkhalterung 43, die hier mehrere Fixierelemente 44 aufweist, die Teil einer Kugelgelenkeinrichtung bilden.

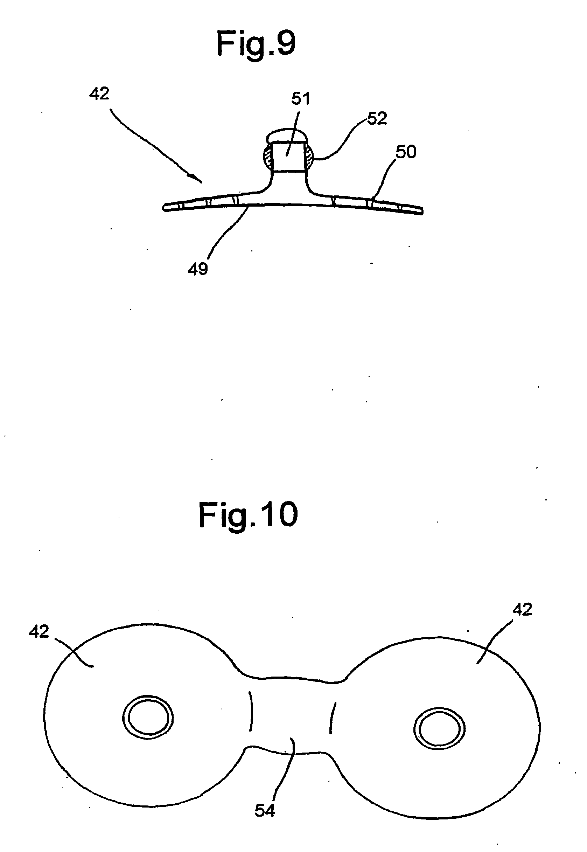

Das Auflageelement 42 weist bei der hier dargestellten Ausführungsform eine tellerartige Gestalt auf und ist aus einem Elastomermaterial, hier volltransparenter Silikonkautschuk gebildet. Das Auflageelernent 42 ist über die Fixierelemente 44 um wenigstens zwei Raumachsen kippbar gelagert. Um eine möglichst leichtgängige Bewegbarkeit des Auflageelementes 42 zu gewährleisten, ist zwischen den Fixierelementen 44 und einem Befestigungsschaftabschnitt (nicht sichtbar) des Auflageelementes 42 ein Ringkörper vorgesehen, der eine sphärische Außenfläche aufweist. (Einzelheiten hierzu werden unter Bezugnahme auf Fig. 9 noch ausführlich erläutert.)

Die Halteeinrichtung 41 umfaßt weiterhin einen Koppelungsabschnitt 45 zur Koppelung der Halteeinrichtung 41 mit einer Atemmaske 46.

Bei der hier dargestellten Ausführungsform ist der Koppelungsabschnitt 45 als ringartiges Element ausgebildet, das unmittelbar auf einen entsprechend komplementär ausgebildeten Anschlußstutzen 47 der Atemmaske 46 aufsteckbar ist. Die Halteeinrichtung 41 ist bei der hier dargestellten Ausführungsform mit einem Befestigungsabschnitt 48 versehen und über diesen mit einem Kopfband verbindbar.

In Fig. 9 ist eine bevorzugte Ausführungsform des Auflageelementes 42 dargestellt. Das Auflageelement 42 ist aus einem elastomeren Material gebildet und weist eine schwach konkav gekrümmte Auflagefläche 49 auf. Das Auflageelement 42 ist mit mehreren feinen Durchgangsbohrungen 50 versehen, durch welche ein Druckausgleich des zwischen dem Auflageelement 42 und der Stirn des Patienten ggf. definierten Zwischenraumes mit der Umgebung erreicht werden kann. Hierdurch wird auf vorteilhafte Weise ein Ansaugen des Auflageelementes 42 an dem Stirnbereich des Patienten verhindert.

Das Auflageelement 42 weist bei der hier dargestellten Ausführungsform einen Schaftabschnitt 51 auf. An diesem Schaftabschnitt 51 ist ein Ringelement 52 vorgesehen, das eine sphärische Außenfläche bildet. Im Zusammenspiel mit diesem Ringelement 52 wird eine vergleichsweise leichtgängige Kugelgelenkeinrichtung geschaffen. Alternativ dazu ist es auch möglich, auf das Ringelement zu verzichten und den entsprechenden sphärischen Abschnitt unmittelbar an dem Schaftabschnitt 51 des Auflageelementes 42 auszubilden.

Bei der in Fig. 10 dargestellten Ausführungsform der Erfindung sind zwei Auflageelemente 42 vorgesehen, die über einen integralen Mittelsteg 54 miteinander verbunden sind. Der Mittelsteg 54 ist derart ausgebildet, daß dieser weiterhin ein Schwenken und Verkippen der beiden Auflageelemente 42 zueinander in einem ausreichenden Winkelbereich zuläßt. Der Abstand der Zentren der beiden Auflageelemente 42 voneinander entspricht vorzugsweise in etwa dem Augenabstand des Maskenanwenders.

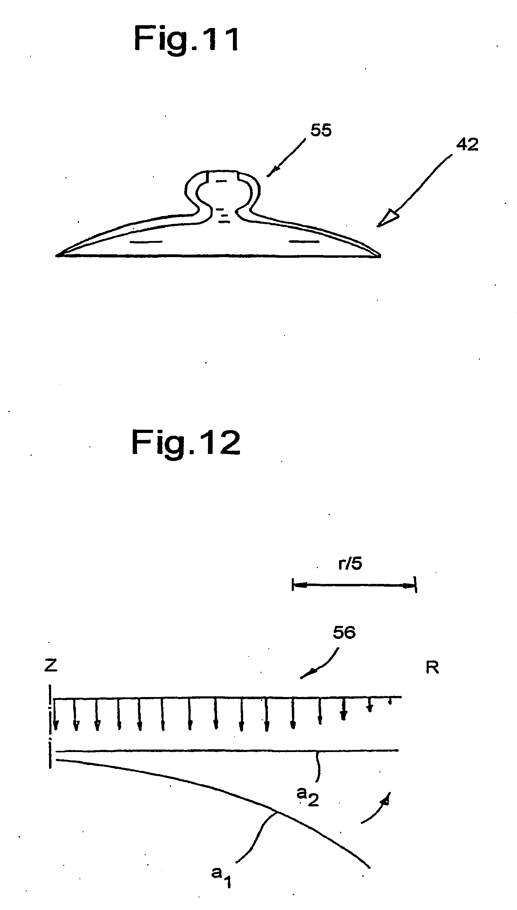

In Fig. 11 ist eine weitere Ausführungsform eines Auflageelementes 42 dargestellt, wobei die Verkippbarkeit des die Auflagefläche bildenden Abschnitts des Auflageelementes über eine Elastomerstruktur 55 erreicht wird, die hier integral mit dem Auflageelement 52 ausgebildet ist. Bei der hier dargestellten Ausführungsform umfaßt die Elastomerstruktur einen im wesentlichen sphärischen Innenraum, der unter vorübergehender elastischer Aufweitung auf einen sphärischen Zapfenabschnitt aufsteckbar ist. Bei der hier dargestellten Ausführungsform weist das Auflageelement ebenfalls einen konkav gewölbten Basiskörper auf, der bei entsprechendem Aufsetzen des Auflageelementes abgeflacht wird.

Die Krümmung des Basiskörpers des Auflageelementes ist vorzugsweise.derart gewählt, daß sich beim Aufsetzen auf eine Ebene eine definierte Flächenpressungsverteilung ergibt. Diese Flächenpressungsverteilung ist beispielhaft in Fig. 12 dargestellt. Die untere, gewölbte Linie al symbolisiert hierbei die Auflagefläche des Auflageelementes in seiner Ausgangsstellung. Die hier gerade dargestellte Linie a2 symbolisiert die in Applikationsposition entsprechend deformierte Auflagefläche des Auflageelementes 42. Im Rahmen der Verformung der Auflagefläche des Auflageelementes ergibt sich die hier vereinfacht durch eine Pfeileschar 56 angedeutete Flächenpressungsverteilung. Die Flächenpressungsverteilung ist hier derart gewählt, daß sich vom Zentrum Z ausgehend zum Randbereich hin zunächst eine im wesentlichen gleichmäßige Flächenpressungsverteilung ergibt, wobei im Bereich von r/5 zum Rand R hin die Flächenpressung allmählich abnimmt.

Die Erfindung ist nicht auf die vorangehend beschriebenen Ausführungsbeispiele beschränkt. Beispielsweise ist es auch möglich, die Stirnauflageelemente abweichend von der hier gewählten tellerartigen Gestalt rechteckförmig oder polygonal auszubilden.

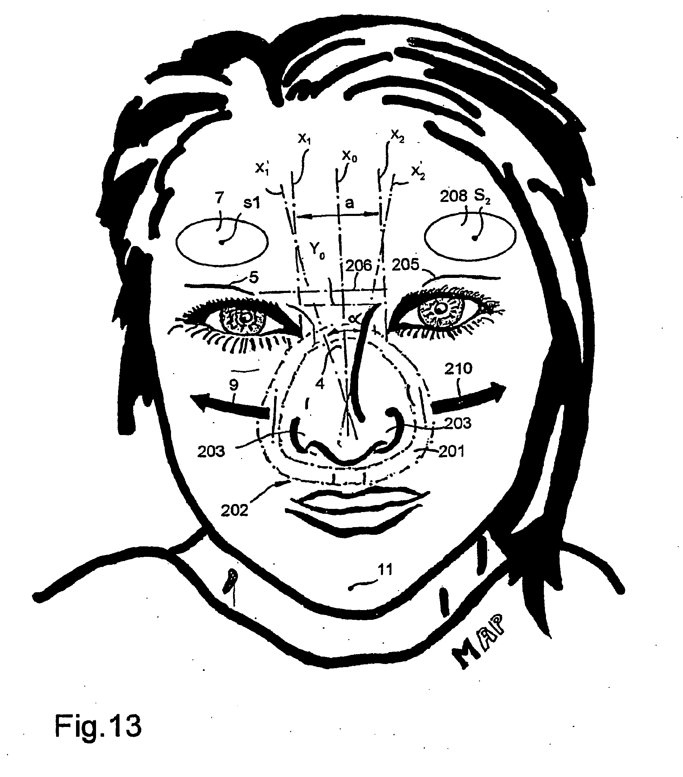

In Figur 13 ist als Frontalansicht die Gesichtsfläche eines Maskenanwenders gezeigt. Zur Applikation einer Atemmaskenanodnung wird diese beispielsweise derart auf das Gesicht des Maskenanwenders aufgesetzt, dass eine, durch eine Dichtlippeneinrichtung der Atemmaskenanordnung und die Gesichtsfläche des Maskenanwenders definierte Dichtlippenauflagezone 201 sich vom Oberlippenbereich 202 ausgehend um die Nasenflügel 203 herum zum Nasenrücken 4 hin erstreckt und diesen vorzugsweise auf Augenhöhe überquert.

Die Atemmaskenanordnung wird über eine, im folgenden noch eingehend erläuterte Stirnauflageeinrichtung, auf dem Stirnbereich des Maskenanwenders abgestützt. Vorzugsweise erfolgt die Abstützung auf dem Stirnbereich über zwei oberhalb einer die Augenbrauen 205 verbindenden Querlinie 206 liegenden Auflagezonen 207, 208. Bei der hier gezeigten Darstellung erfolgt die Abstützung der Atemmaskenanordnung im Stirnbereich an zwei Auflagezonen 207, 208, wobei der Abstand der Flächenschwerpunkte S1, S2 der Auflagezonen 207, 208 vorzugsweise in etwa dem Augenabstand des Maskenanwenders entspricht. Der Auflagedruck der Dichtlippeneinrichtung der Atemmaskenanordnung im Bereich der Dichtlippenauflagezone 201 ist in einstellbarer Weise veränderbar, indem zur Auflage in den Auflagezonen 207, 208 vorgesehenen Auflageelemente, um Schwenkachsen X0, X1, X1', X2, X2' sowie vorzugsweise auch um eine Querachse Y0 verschwenkbar sind.

Die Abstützung der Atemmaskenanordnung auf dem Gesicht des Maskenanwenders kann durch die nachfolgend noch näher beschriebene Applikationsvorrichtung derart vorgenommen werden, dass die Atemmaskenanordnung und die Stirnauflageeinrichtung im wesentlichen an drei voneinander beabstandeten Zonen auf dem Gesicht des Maskenanwenders abgestützt sind. Im Stirnbereich stützt sich dabei die Stirnauflageeinrichtung an den beiden Auflagezonen 207,208 ab. Die Atemmaskenanordnung stützt sich über die Dichtlippenauflagezone 201 auf dem Gesicht des Maskenanwenders ab. Durch die Abstützung der Applikationsvorrichtung und der Atemmaskenanordnung auf dem Gesicht des Maskenanwenders an drei Haupt-Tragezonen ergibt sich in vorteilhafter Wiese eine statisch bestimmte Abstützung der Atemmaske. Die zur Halterung der Stirnauflageeinrichtung im Stirnbereich erforderlichen Haltekräfte werden vorzugsweise über eine obere Kopfbandanordnung aufgebracht. Zur Fixierung der Atemmaskenanordnung im Nasenbereich ist vorzugsweise eine untere Gurtbandanordnung vorgesehen, über welche die Atemmaskenanordnung durch beidseitig angreifende, seitlich zu den Wangen gerichtete Zugkräfte 209, 210 gegen die Gesichtsfläche des Maskenanwenders gedrängt wird. Die Zugkräfte 209, 210 werden vorzugsweise durch eine untere, um den Hinterkopfbereich des Maskenanwenders herumgeführte untere Gurtbandanordnung aufgebracht.

Die eine Einstellung der Abstütz-Konfiguration der Stirnauflageeinrichtung ermöglichenden Schwenkachsen X0, X1, X1', X2, X2', verlaufen vorzugsweise vom Stirnbereich zum Oberlippenbereich 202 des Maskenanwenders hin gerichtet. Die hier eingetragenen Achsen X1, X2 sind zueinander parallel ausgerichtet und voneinander um einen Abstand a beabstandet, der im wesentlichen der Breite des Nasenrückens, insbesondere der Breite des Nasenrückens im Bereich der Tränenkanalmündungen des Maskenanwenders entspricht.

Die hier zueinander geneigten Schwenkachsen X1', X2', weisen auf Höhe der Auflagezone 207, 208 ebenfalls vorzugsweise ein Abstandsmaß im Bereich der Nasenrückenbreite a auf. Die Achsen X1', X2' sind zueinander um einen Winkel α geneigt, der vorzugsweise derart gewählt ist, dass sich die beiden Achsen im Bereich zwischen der Nasenwurzel und dem Kinn 211 des Maskenanwenders schneiden.

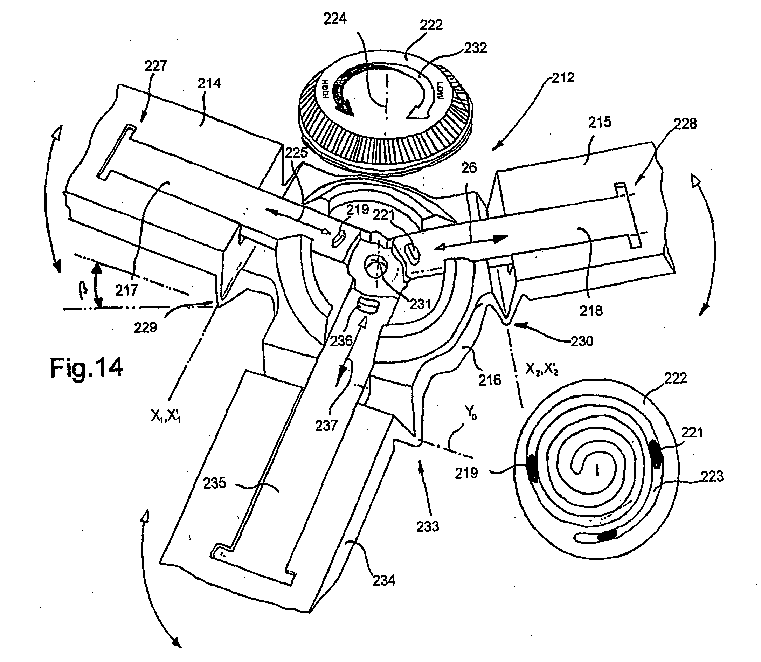

In Fig. 14 ist in Form einer perspektivischen Darstellung ein Stelltrieb 212 dargestellt, über welchen die Schwenkposition eines linken Armelementes 214 und eines rechten Armelementes 215 gegenüber einem Basisabschnitt 216 in einstellbarer Weise veränderbar ist.

Der Stelltrieb 212 umfasst hierbei ein dem linken Armelement 214 zugeordnetes Kopplungsorgan 217 sowie ein dem rechten Armelement 215 zugeordnetes Koppelungsorgan 218. Jedes der beiden Koppelungsorgane 217, 218 ist mit wenigstens einem Eingriffsabschnitt 219, 221 versehen, der mit einem hier abgehoben dargestellten Stellrad 222 über eine an der Unterseite des Stellrades 222 ausgebildete Spiralstruktur 223 (s. Skizze rechts unten) in Eingriff steht. Durch Drehung des Stellrades 222 um dessen Rotationsachse 224 werden die Eingriffsabschnitte 219, 221 wie durch die Pfeilsymbole 225, 226 angedeutet, gemeinsam mit den Koppelungsorganen 217, 218 in radialer Richtung verschoben.

Die Koppelungsorgane 217, 218 sind bei dem dargestellten Ausführungsbeispiel bewegbar mit dem ihm zugeordneten linken bzw. rechten Armelement 214, 215 gekoppelt. Bei dem Ausführungsbeispiel erfolgt die Koppelung der Koppelungsorgane 217, 218 mit den ihnen zugeordneten Armelementen 214, 215 jeweils über eine Gelenkabschnitt 227 bzw. 228, der eine Schwenkbewegung des Koppelungsorgans 217, 218 gegenüber dem ihm zugeordneten Armelement 214, 215 ermöglicht. Die Armelemente 214, 215 sind mit dem Basisabschnitt 216 über eine Gelenkverbindung 229, 230 gekoppelt. Die Gelenkverbindungen 229, 230 sind hier als Filmscharniere ausgeführt. Die Armelemente 214, 215 sind hierbei integral mit dem Basisabschnitt 216 ausgeführt. Durch die Gelenkverbindungen 229, 230 werden die vorangehend in Verbindung mit Fig. 13 angesprochenen Gelenkachsen X1, X1' bzw. X2, X2' definiert. In Folge des, im Bereich der Gelenkabschnitte 227, 228 sowie der Gelenkverbindungen 229, 230 in das jeweilige Armelement eingeleiteten Kräftepaares, kann das jeweilige Armelement gegenüber dem Basisabschnitt 216, in eine durch den Radialabstand des Eingriffsabschnitts 219, 221 von der Rotationsachse 224 bestimmte Schwenkposition gebracht werden. Der maximale Radialhub der Eingriffsabschnitte 219, 221 sowie die räumliche Lage der Gelenkabschnitte und der Gelenkverbindungen 227, 228, 229, 230, sind derart gewählt, dass beispielsweise in einem Drehbereich des Stellrades 222 um einen Drehwinkel von 540°, ein Schwenkwinkel β der Armelemente 214, 215 im Bereich von 0 bis 40° einstellbar ist.

Das Stellrad 222 ist aus einem Kunststoffmaterial gefertigt und über einen zentralen Drehzapfen (hier nicht sichtbar) in eine, in dem Basisabschnitt 216 ausgebildete Bohrung 231 eingesetzt. Das Stellrad 222 weist hier einen Durchmesser von 45 mm auf und ist auf seiner Aussenseite mit einer Markierung 232 versehen, welche die durch Drehung des Stellrades 222 erreichte Einstellwirkung verdeutlicht.

Bei dem hier gezeigten Ausführungsbeispiel ist der Basisabschnitt 216 über eine weitere Gelenkverbindung 233 mit einem Halterungsabschnitt 234 gekoppelt. Über die hier gezeigte Gelenkverbindung 233 wird die in Fig. 13 angedeutete Schwenkachse Y0 festgelegt. Die Schwenkbewegung des Halterungsabschnittes 234 gegenüber dem Basisabschnitt 216 wird in gleicher Weise wie vorangehend bezüglich der linken und rechten Armelemente 214, 215 über ein Koppelungsorgan 235 festgelegt das über einen Eingriffsabschnitt 236 mit dem Stellrad 222 in Eingriff steht und wie durch das Pfeilsymbol 237 angedeutet, gegenüber der Rotationsachse 224, in radialer Richtung bewegbar ist. Der Halterungsabschnitt 34 ist über eine hier nicht näher dargestellte Zwischenstruktur mit einem Maskenbasiskörper einer Atemmaskenanordnung verbunden.

An den linken und rechten Armelementen 214, 215 sind wie im folgenden noch beschrieben werden wird, Auflage-Pads angebracht, über welche sich die Armelemente 214, 215 an den Auflagezonen 207, 208 (Fig. 13) des Maskenanwenders abstützen.

Die Armelemente 214, 215 können wie ebenfalls im folgenden noch erläutert werden wird, vorzugsweise über eine obere Kopfbandanordnung auf den Stirnbereich des Maskenanwenders gezogen werden.

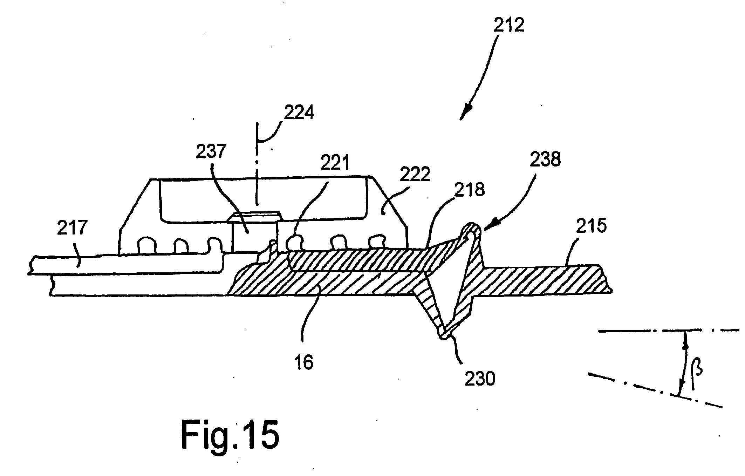

In Fig. 15 ist eine weitere Variante des Stelltriebs 212 dargestellt, bei welchem ein radial zur Rotationsachse 224 des Stellrades 222 bewegbares Koppelungsorgan 218 einstückig mit dem ihm zugeordneten Armelement 215 ausgebildet ist, wobei eine hinreichende Schwenkbewegbarkeit über eine Filmscharnierstruktur 238 erreicht wird. Der Basisabschnitt 216 ist ebenfalls einstückig mit dem Armelement 215 ausgebildet und mit diesem ähnlich wie bei dem Ausführungsbeispiel gemäß Fig. 14 über eine Gelenkverbindung 230, die ebenfalls als Filmscharnier ausgebildet ist, gekoppelt. Die durch die Filmschamierstruktur 238 und die Gelenkverbindung 230 definierten Krafteinleitungsabschnitte sind derart voneinander beabstandet, dass infolge des durch das Stellrad 222 einstellbaren Radialhubes des Koppelungsorganes 218 ein ausreichender Schwenkwinkel β des Armelementes 215 erreicht werden kann.

Das Stellrad 222 ist an einem vom Basisabschnitt 216 aufragenden Drehzapfen 239 drehbewegbar gelagert. Auf der, dem Basisabschnitt 216 zugewandten Unterseite des Stellrades 222 sind die wie bei dem vorangehend beschriebenen Ausführungsbeispiel Spiralstrukturen ausgebildet, die mit dem integral mit dem Koppelungsorgan 218 ausgebildeten Eingriffsabschnitt 221 in Eingriff stehen.

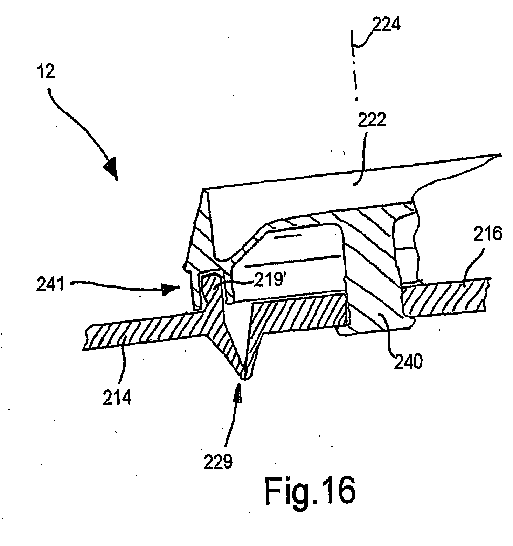

In Fig. 16 ist eine weitere Ausführungsform des Stelltriebs 212 ausgeführt. Der Stelltrieb 212 umfasst auch hier ein Stellrad 222 das über einen Drehzapfen 240 drehbewegbar mit dem Basisabschnitt 216 gekoppelt ist. Auf der, dem Basisabschnitt 216 zugewandten Unterseite des Stellrades 222 ist eine Spiralstruktur 241 ausgebildet, die unmittelbar mit einem Eingriffsabschnitt 219' in Eingriff steht, der an dem Armelement 214 ausgebildet ist. Das Armelement 214 ist über eine, hier wiederum als Filmscharnier ausgebildete Gelenkverbindung 229 mit dem Basisabschnitt 216 schwenkbewegbar gekoppelt. Durch Drehung des Stellrades 222 um die durch den Drehzapfen 214 definierte Rotationsachse 224 können unterschiedliche Abstände des Eingriffsabschnittes 219' von der Rotationsachse 224 eingestellt werden, wodurch das Armelement 214 gegenüber dem Basisabschnitt 216 in jeweils gewünschte Schwenkpositionen schwenkbar ist.

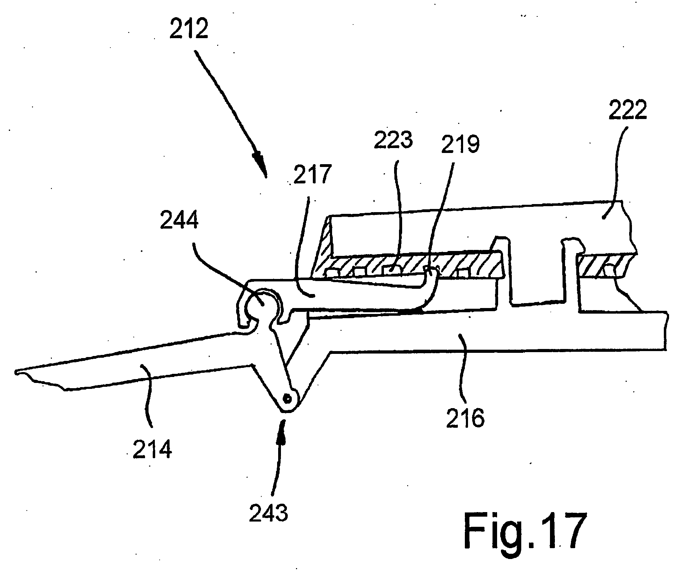

In Fig. 17 ist eine weitere Ausführungsform eines erfindungsgemäßen Stelltriebes 212 dargestellt, bei welchem der Basisabschnitt 216 und das Armelement 214 als separate Teile ausgebildet sind, die über eine Scharniereinrichtung 243 miteinander gekoppelt sind. An dem Armelement 214 ist ein Knauf 244 ausgebildet, an welchem das Koppelungsorgan 217 schwenkbewegbar angelenkt ist. Das Koppelungsorgan 217 weist ähnlich wie bei den vorangehend beschriebenen Ausführungsformen einen Eingriffsabschnitt 219 auf, der mit dem Stellrad 222 über eine Spiralstruktur 223 in Eingriff steht. Durch Drehung des Stellrades 222 kann das Koppelungsorgan 217 in radialer Richtung definiert verschoben werden und schwenkt dabei das Armelement um die Scharniereinrichtung 243 in eine definierte Winkelstellung gegenüber dem Basisabschnitt 216. Es ist möglich, Bremsmittel vorzusehen, durch welche das Stellrad 222 in der gewünschten Drehposition hinreichend fest fixiert ist. Derartige Bremseinrichtungen können beispielsweise durch feine Rastvorsprünge verwirklicht sein, die im Rahmen einer durch die Eigenelastizität der Bauteile ermöglichten Relativbewegung der zueinander bewegten Komponenten, in Eingriff bzw. außer Eingriff gelangen. Es ist auch möglich, die Spiralstruktur derart auszubilden, dass diese im wesentlichen selbsthemmend ist.

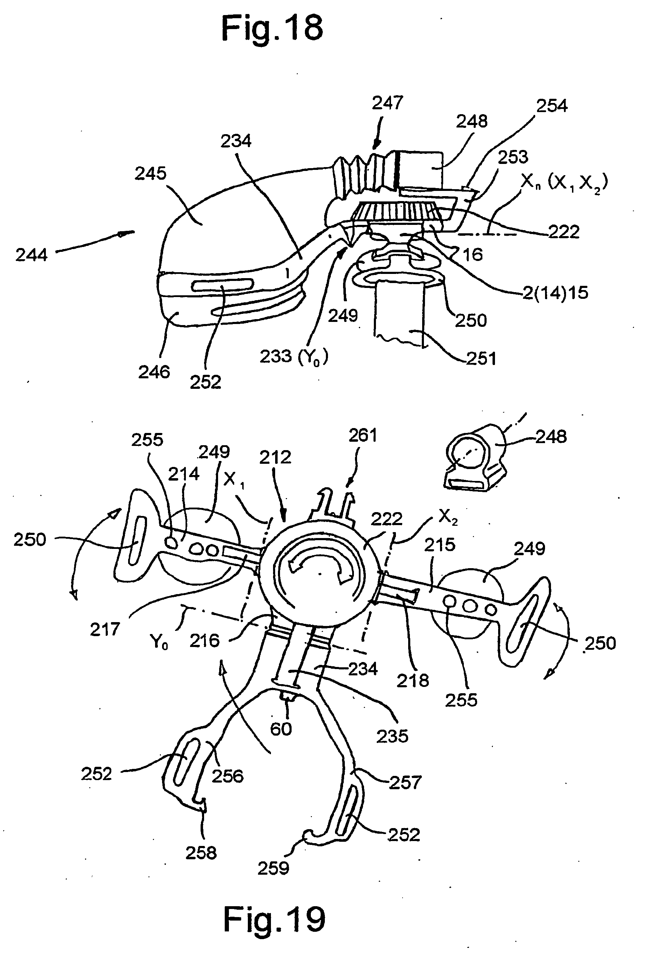

In Fig. 18 ist eine erfindungsgemäße Applikationsvorrichtung mit darin aufgenommener Atemmaskenanordnung 244 dargestellt. Die Atemmaskenanordnung 244 umfasst einen Maskenbasiskörper 245 und eine mit diesem gekoppelte Dichtlippeneinrichtung 246. Die Dichtlippeneinrichtung 246 ist aus einem Elastomermaterial vorzugsweise volltransparentem Silikonkautschuk gefertigt und kontaktiert in Applikationsposition in abdichtender Weise die in Fig. 13 dargestellte Dichtlippenauflagezone 201 des Maskenanwenders. Die Zuleitung des Atemgases in den durch den Maskenbasiskörper 245 definierten Maskeninneraum erfolgt über einen flexiblen Leitungsabschnitt 247, der bei dem gezeigten Ausführungsbeispiel ebenfalls aus einem elastomeren Material gefertigt und als Faltenbalgstruktur ausgebildet ist. An den flexiblen Leitungsabschnitt schließt sich ein Atemschlauch-Anschlussstutzen 248 an der einen Innendurchmesser im Bereich von 12 bis 35 mm aufweist. Der Atemschlauch-Anschlussstutzen 248 ist mit dem Basisteil 216 der Stirnauflageeinrichtung gekoppelt. Die Stirnauflageeinrichtung 216 umfasst das Stellrad 222, das sich hier in einem Zwischenbereich zwischen dem flexiblen Leitungsabschnitt 247 bzw. dem Atemschlauch-Anschlussstutzen 48 und dem Basisabschnitt 216 befindet. Das Stellrad 222 kann bei Umgreifen des flexiblen Leitungsabschnittes 247 über die Fingerspitzen des Daumens und des Zeigefingers des Maskenanwenders von diesem gedreht werden. Durch Drehen Stellrades 222, ist es möglich, die Armelement 214, 215 um die Schwenkachse Xn zu schwenken. Hierdurch wird es möglich, ein, zur Auflage auf dem Stirnbereich des Maskenanwenders vorgesehenes Stirn-Pad 249 gegenüber dem Basisabschnitt 216, in einstellbarer Weise zu positionieren. An dem Armelement 215 (214) ist ein Ösenabschnitt 250 ausgebildet, durch welchen ein Abschnitt einer oberen Kopfbandanordnung 251 hindurchgeführt ist.

Bei der hier dargestellten Ausführungsform ist der Basisabschnitt 216 über die Gelenkverbindung 233 schwenkbewegbar mit einem Halterungsabschnitt 234 verbunden. Der Halterungsabschnitt 234 ist rahmenartig ausgebildet und umgreift den Maskenbasiskörper 245 zumindest in dessen Seitenbereich. An dem Halterungsabschnitt 234 sind ebenfalls Ösenabschnitte 250 ausgebildet, durch welche ein Abschnitt einer unteren Kopfbandanordnung (nicht dargestellt) hindurch geführt werden kann. Alternativ zu der Ausbildung der genannten Ösenabschnitte 252 oder auch in Kombination hiermit, ist es möglich, andere Koppelungsstrukturen zur Koppelung des Halterungsabschnittes 234 oder des Maskenbasiskörpers 245 mit einer Kopfbandanordnung vorzusehen.

Das Schwenken des Basisabschnitts 216 um die durch die Gelenkverbindung 233 definierte Schwenkachse Y0 erfolgt bei dieser Ausführungsform simultan mit dem Schwenken der Armteile 214, 215 um die Achsen X1, X2.

Der Atemschlauch-Anschlussstutzen 248 ist mit dem Basisabschnitt 216 gekoppelt. Beim vorliegenden Ausführungsbeispiel erfolgt die Koppelung über einen an dem Stellrad 222 vorbei geführten Haltefuß 253. Der Haltefuß 253 ist auf einen Steckverbindungsabschnitt des Basisteils 216 aufgesteckt und mit einer Rasteinrichtung 254 versehen, durch welche ein, mit dem Atemschlauch-Anschlussstutzen 248 gekoppelter Endabschnitt einer Atemgasleitung, zusätzlich fixiert ist. Die zur Auflage auf der Stirn des Maskenanwenders vorgesehenen Stirn-Pads 249 sind an dem jeweiligen Armelement 214, 215 verschiebbar angebracht.

In Fig. 19 ist eine Applikationsvorrichtung für eine Atemmaske dargestellt, bei welcher das linke Armelement 214, das rechte Armelement 215 und der Halterungsabschnitt 234 über den mit einem Stellrad 222 betätigbaren Stelltrieb 212 definiert um die Achsen X1, X2 bzw. Y0 kippbar sind. Das Kippen der genannten Teile erfolgt jeweils über die, diesen Teilen zugeordneten Koppelungsorgane 217, 218 und 235. Die Abstützung der Armelemente 214, 215 auf dem Stirnbereich eines Maskenanwenders erfolgt über die bereits in Verbindung mit Fig. 18 genannten Stirn-Pads 249, die bei dieser Ausführungsform in Einsatzöffnungen 255 einsteckbar sind, die an dem jeweiligen Armelement 214, 215 ausgebildet sind.

Der Halterungsabschnitt 234 ist hier aus einem hochfesten Kunststoffmaterial, vorzugsweise Polyamid gefertigt und rahmenartig ausgebildet. Der Halterungsabschnitt 234 umfasst zwei Haltearme 256, 257 die jeweils mit einem Ösenabschnitt 252 versehen sind, durch welchen ein Endabschnitt einer unteren Gurtbandanordnung hindurchgeführt werden kann.

Die Haltearme 256, 257 sind mit einer Eingriffsstruktur 258, 259 versehen über welche die Haltearme mit einem Maskenbasiskörper in Eingriff bringbar sind. Die Fixierung des Maskenbasiskörpers in dem Halterungsabschnitt 234 erfolgt weiterhin durch einen Rastvorsprung 260 der integral mit dem Halterungsabschnitt 234 ausgebildet ist. Auf einer, dem Halterungsabschnitt 234 abgewandten Seite des Basisabschnitts 216 ist ein Aufsteckabschnitt 261 ausgebildet, auf welchen ein Aternschlauch-Anschlussstutzen aufsteckbar ist. Dieser Atemschlauch-Anschlussstutzen 248 kann wie in Fig. 18 angedeutet, zur Weiterleitung des Atemgases in einen, durch einen Maskenbasiskörper definierten Maskeninnenraum dienen.

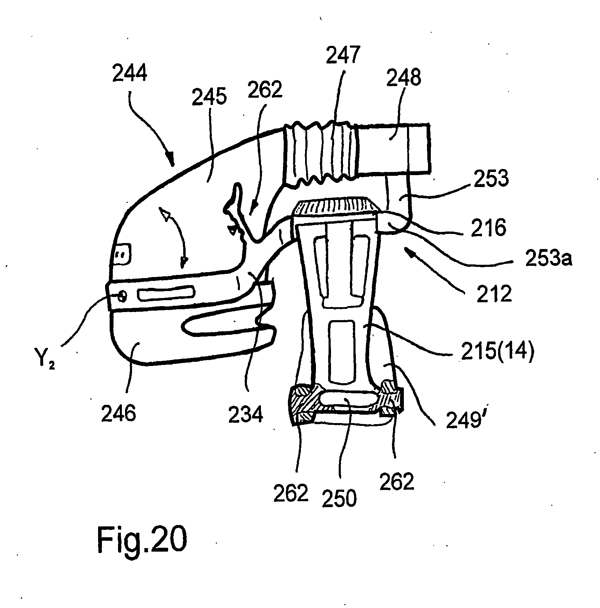

In Fig. 20 ist eine, mit einer Atemmaskenanordnung 244 versehene Applikationsvorrichtung dargestellt, die ähnlich wie die vorangehend beschriebenen Ausführungsformen einen Stelltrieb 212 umfasst, über welchen die beidseitig von einem Basisabschnitt 216 des Stelltriebs 212 abragenden Armelemente 214, 215 in einstellbarer Weise geschwenkt werden können. Abweichend von den vorangehend beschriebenen Ausführungsformen, ist hier der Maskenbasiskörper 245 unabhängig von der durch den Stelltrieb 212 veranlassten Einstellbewegung der Armelemente um eine Schwenkachse Y1 kippbar, die sich in etwa im Bereich der den Nasenflügeln eines Maskenanwenders benachbarten Zone der Dichtlippeneinrichtung erstreckt und die im wesentlichen parallel zu einer die Augenbrauen verbindenden Querlinie 206 erstreckt. Die Schwenkposition des Maskenbasiskörpers 245 gegenüber dem Halterungsabschnitt 234 ist durch eine Aretiereinrichtung 262 festlegbar. Eine hinreichende Relativbewegbarkeit des Maskenbasiskörpers 245 gegenüber einem zum Anschluss einer Atemgasleitung vorgesehenen Atemschlauch-Anschlussstutzen 248 wird durch einen flexiblen Leitungsabschnitt 247 erreicht, wie er auch bei dem Ausführungsbeispiel gemäß Fig. 18 vorgesehen ist. Bei diesem Ausführungsbeispiel ist der Maskenbasiskörper vorzugsweise integral mit einer Dichtlippeneinrichtung 46 aus einem elastomeren Material, vorzugsweise Silikonkautschuk gefertigt. Der Atemschlauch-Anschlussstutzen 248 ist bei diesem Ausführungsbeispiel starr mit dem Basisabschnitt 216 gekoppelt und aus einem thermoplastischen Hart-Kunststoff gefertigt.

An den Armelementen 214, 215 sind ebenfalls zur Auflage auf der Stirnfläche des Maskenanwenders vorgesehene Stirn-Pads 249' angebracht, wobei diese Stirn-Pads 249' kippbewegbar an Zapfen 262 eingehängt sind, die integral mit dem jeweiligen Armelement 215, 214 ausgebildet sind. Zwischen diesen Zapfen 262 befindet sich wiederum ein Ösenabschnitt 250 zur Durchführung eines Endabschnittes einer oberen Gurtbandanordnung. Es ist möglich, den Basisabschnitt 216 verschiebbar auf einem sich vom Halterungsabschnitt 234 zu dem Haltefuß 253 hin erstreckenden Stegabschnitt 253a zu führen. Hierdurch wird es möglich, auch den Vertikalabstand der Stirnauflageeinrichtung von der Dichtlippeneinrichtung 246 in einstellbarer Weise zu verändern.

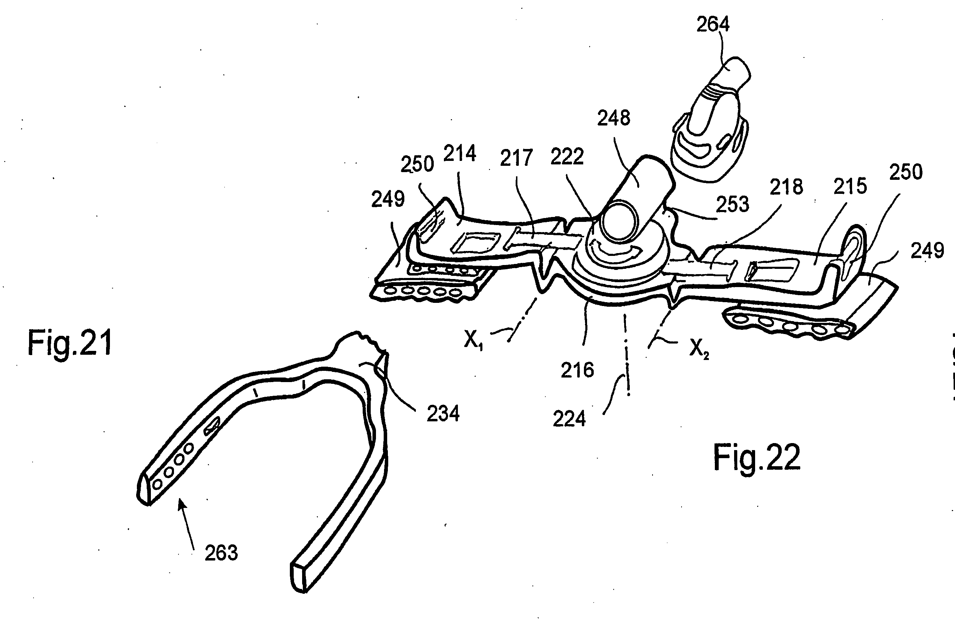

In Fig. 21 ist eine weitere Ausführungsform des Halterungsabschnittes 234 dargestellt wie er beispielsweise bei den Ausführungsformen nach den Fig. 19 und 20 Anwendung finden kann. Der Halterungsabschnitt 234 ist mit einer, zur Ankoppelung eines Maskenbasiskörpers (hier nicht dargestellt) vorgesehen Koppelungsstruktur versehen, durch welche der Maskenbasiskörper mit dem Halterungsabschnitt 234 in unterschiedlichen Koppelungspositionen koppelbar ist.

In Fig. 22 ist eine Stirnauflageeinrichtung dargestellt, die über einen Atemschlauch-Anschlussstutzen 248 auf einen Anschlussabschnitt 264 einer hier angedeutet Atemmaske aufsteckbar ist.

Der Atemschlauch-Anschlussstutzen 248 ist mit einem Basisabschnitt 216 der Stirnauflageeinrichtung über einen Haltefuß 253 gekoppelt. Über ein, zwischen dem Atemschlauch-Anschlussstutzen 248 und dem Basisabschnitt 216 angeordnetes Stellrad 222, sind die Koppelungsorgane 217, 218, in einstellbarer Weise in einer Richtung radial zur Rotationsachse 24 des Stellrades 222 bewegbar. Durch entsprechende Positionierung der Koppelungsorgane 217, 218 werden die Armelemente 214, 215 um die Achsen X1, X2 definiert geschwenkt.

An den Armelementen 214, 215 sind Stimauflage-Pads 249 angebracht, über welche die Armelemente 214, 215 sich auf dem Stirnbereich eines Maskenanwenders abstützen. Im Bereich der Stirnauflage-Pads 249 sind an den Armelementen 214, 215 Ösenabschnitte 250 ausgebildet, durch welche der jeweilige Endabschnitt einer oberen Gurtbandanordnung hindurchgeführt werden kann.

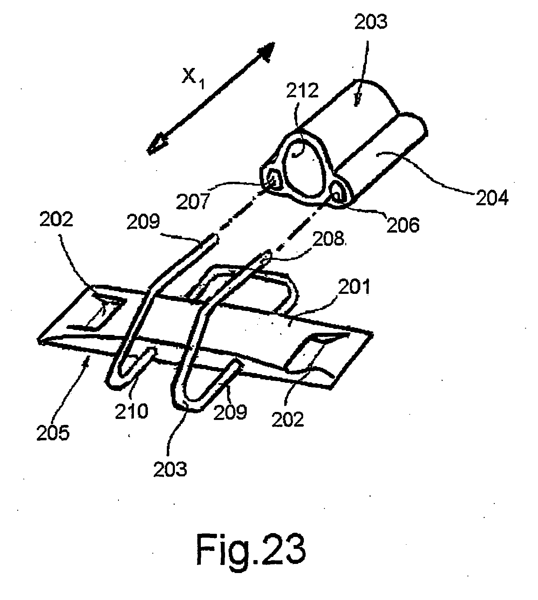

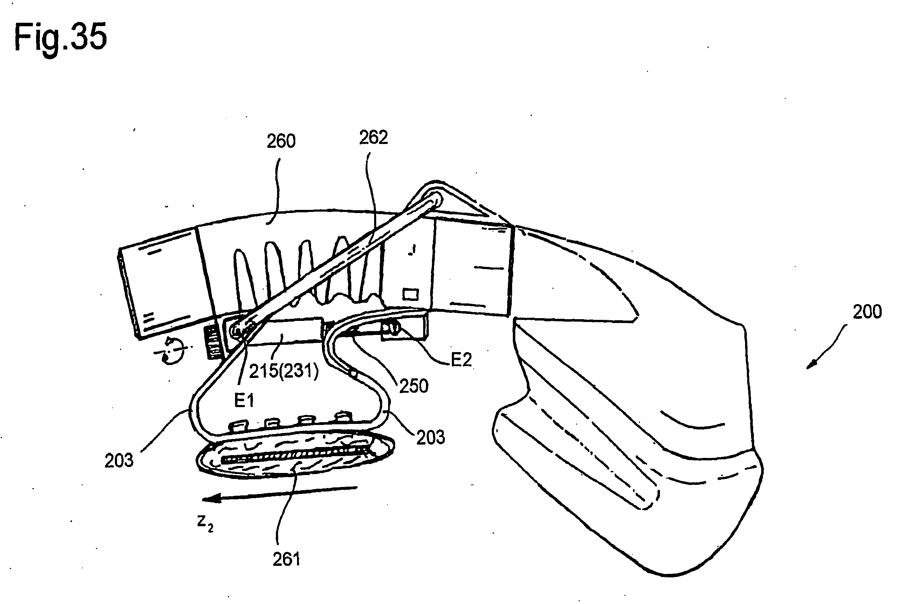

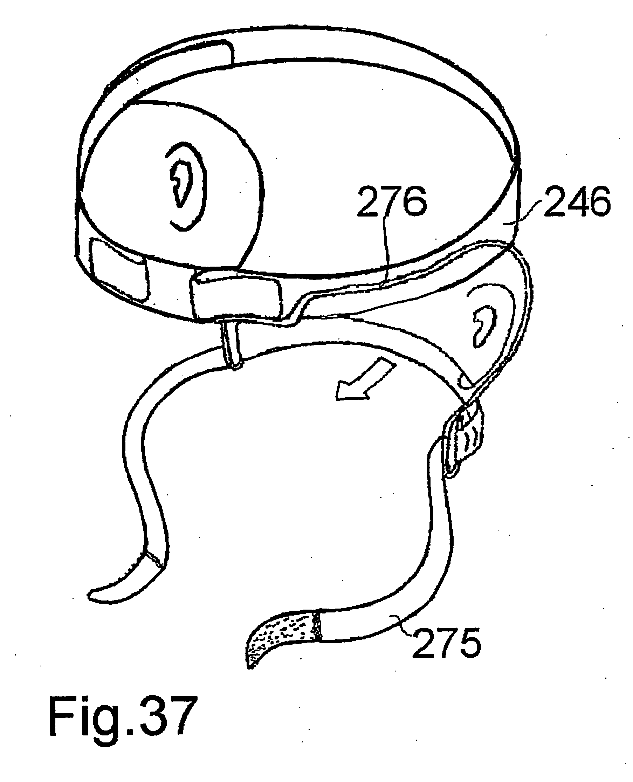

Figur 23 zeigt eine Stirnauflageeinrichtung, die beispielsweise in Verbindung mit der in Figur 35 gezeigten Atemmaske 200 verwendet werden kann. Diese Stirnauflageeinrichtung umfasst einen aus einem Weich-Material insbesondere Elastomer-Material gefertigten Stirnauflageabschnitt 201, der über einen oberen Kopfbandabschnitt einer Kopfbandanordnung ( siehe hierzu Figur 37 ) im Stirnbereich eines Maskenanbinders applizierbar ist. Der Stirnauflageabschnitt 201 umfasst bei diesem Ausführungsbeispiel zwei Laschenabschnitte 202, durch die ein Fixierbandabschnitt der Kopfbandanordnung hindurchführbar ist. An dem Stirnauflageabschnitt 201 ist eine Koppelungsstruktur 203 angebracht, über welche ein Leitungsverbindungselement 204 in einem durch die Geometrie der Koppelungsstruktur bestimmten Abstand vor der unteren Auflagefläche 205 des Stirnauflageabschnitts abgestützt ist. Das Leitungsverbindungselement ist bei diesem Ausführungsbeispiel ebenfalls aus einem Elastomer-Material ausgebildet und umfasst zwei Fügeöffnungen 206, 207, über welche das Leitungsverbindungselement 204 hinreichend festsitzend auf zwei Schenkelabschnitte 208, 209 der Koppelungsstruktux 203 aufsetzbar ist.

Das Leitungsverbindungselement 204 und die Koppelungsstruktur 203 können derart ausgebildet sein, dass das Leitungsverbindungselement 204 in Richtung der hier angedeuteten Achse X 1 stufenlos relativ zu dem Stirnauflageabschnitt 201 positionierbar ist. Weitere Einstellmöglichkeiten der Position des Leitungsverbindungselements 204 relativ zu dem Stirnauflageabschnitt 201 können auch durch Bereitstellung unterschiedlicher Koppelungsmöglichkeiten der Koppelungsstruktur 203 mit dem Stirnauflageabschnitt 201 bewerkstelligt werden. Bei dem hier dargestellten Ausführungsbeispiel ist es möglich, die hier den Stirnauflageabschnitt 201 paarweise durchsetzenden Längsschenkel 209, 210 verschiebbar in dem Stirnauflageabschnitt 201 zu lagern. Die Abmessungen der Längsschenkel 209, 210 und der in dem Stirnauflageabschnitt, zur Durchführung dieser Längsschenkel vorgesehenen Durchgangskanäle sind vorzugsweise derart festgelegt, dass eine hinreichend reibschlüssige Fixierung der Koppelungsstruktur 203 in der gewünschten Relativposition gewährleistet ist.

Die Koppelungsstruktur 203 bildet einen Durchgangskanal 212, der derart bemessen ist, dass in diesen ein Anschlussstutzenabschnitt einer Atemmaske ( vergleiche hierzu Figur 35 ) in diesen einsteckbar ist. Der Durchgangskanal 212 ist weiterhin derart dimensioniert, dass in diesen auf einer der Atemmaske abgewandten Seite ein Atemgasschlauch insbesondere über eine Auswasch-Ventilanordnung einsteckbar ist.

Der entsprechend der individuellen Gesichtstektur des Maskenanwenders erforderliche Abstand zwischen der Auflagefläche 205 des Stirnauflageabschnitts 201 und der Koppelungsstruktur 203 kann durch Bereitstellung verschiedener Koppelungsstrukturen 203 oder auch durch plastische Umformung, zum Beispiel Verbiegen der Koppelungsstruktur 203 eingestellt werden.

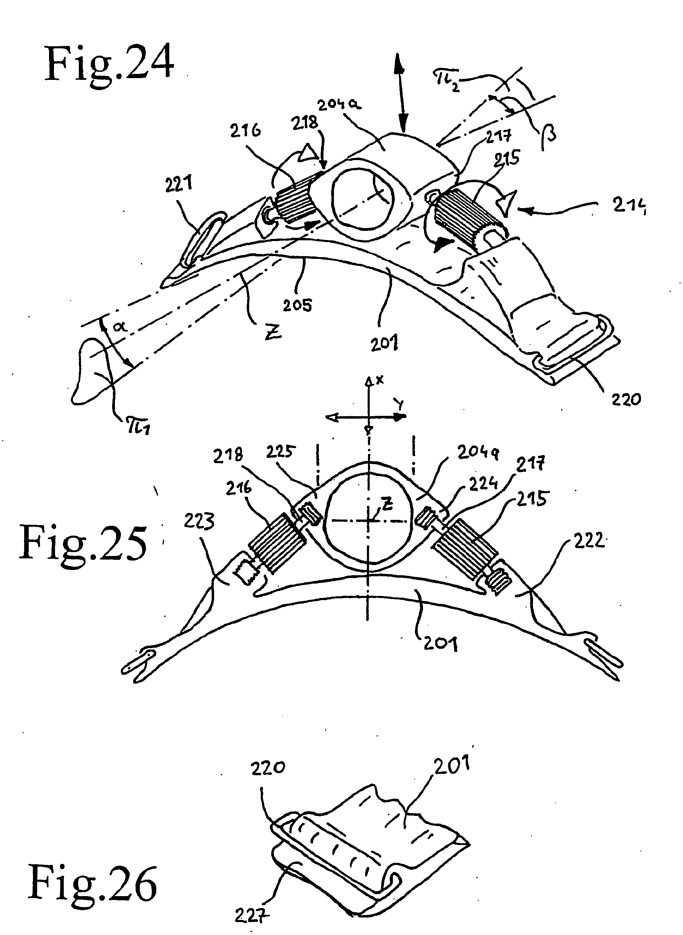

In Figur 24 ist ein weiteres Ausführungsbeispiel einer Stirnauflageeinrichtung dargestellt. Diese Stirnauflageeinrichtung umfasst ebenfalls einen Stirnauflageabschnitt 201, der aus einem hinreichend elastisch verformbaren Material vorzugsweise Silikonkautschuk gefertigt ist. An diesem Stirnauflageabschnitt 201 ist ein Leitungsdurchführungselement 204 a über eine Einstelleinrichtung 214 verstellbar abgestützt. Das Leitungsdurchführungselement 204 a kann ähnlich wie vorangehend in Verbindung mit Figur 23 beschrieben als Verbindungselement ausgebildet sein, über welches eine Koppelung eines maskenseitig vorgesehenen Anschlussstutzens mit einer Atemgasleitung ermöglicht ist. Alternativ hierzu ist es auch möglich, das Leitungsdurchführungselement 204 derart auszubilden, dass dieses einen Atemgasleitungsabschnitt lediglich umgreift und diesen dabei höhenverstellbar mit dem Stirnauflageabschnitt 201 koppelt.

Die Einstelleinrichtung 214 umfasst bei diesem Ausführungsbeispiel zwei Stelltriebeinrichtungen 215, über welche der Abstand der Zentralachse Z des Leitungsdurchführungselements 204 a gegenüber der Auflagefläche 205 des Stirnauflageabschnitts 201 stufenlos einstellbar ist. Die Stelltriebeinrichtungen 215, 216 sind bei diesem Ausführungsbeispiel mit Innengewindebuchsen versehen, die über eine außen umfangsseitig vorgesehene Rändelung manuell drehbar sind. Die Innengewindebuchsen stehen mit hier nicht näher dargestellten Gewindeabschnitten von Stellschrauben 217, 218 in Eingriff, sodass durch manuelle Drehung der jeweiligen Innengewindebuchse die wirksame Länge einer durch die Innengewindebuchse und die damit in Eingriff stehende Stellschraube, gebildeten Strebe stufenlos einstellbar ist. Durch Veränderung der wirksamen Länge dieser Streben kann auch der Abstand des Leitungsverbindungselements 204 a vom Maskenanwender individuell eingestellt werden.

An dem Stirnauflageabschnitt 201 sind ebenfalls Koppelungsmöglichkeiten zur Koppelung des Stirnauflageabschnitts mit einer Kopfbandanordnung vorgesehen. Bei diesem Ausführungsbeispiel sind diese Koppelungsmöglichkeiten durch Bügelelemente 220, 221 gegeben.

Wie aus Figur 25 hervorgeht, ist der Stirnauflageabschnitt 201 mit Abstützstellen 222, 223 versehen, in welchen die beiden Stelltriebeinrichtungen 215, 216 jeweils einseitig verankert sind. An dem Leitungsdurchführungselement 204 a sind ebenfalls Abstützstellen 224 ausgebildet, in welchen die Endabschnitte der Stellschrauben 217, 218 hinreichend drehfest verankert sind.

Die Anordnung ist hier derart getroffen, dass durch Betätigung der beiden Stelltriebeinrichtungen 215, 216 die wirksame Länge der das Leitungsdurchführungselement 204 a mit den jeweiligen Abstützstellen 222, 223 koppelnden Struktur stufenlos veränderbar ist. Durch Drehung der hier angedeuteten, außen umfangsseitig gerändelten Innengewindebuchsen ( siehe Pfeilsymbole in Figur 24 ) ist es möglich, die Position des Leitungsdurchführungselements 204 a relativ zu dem Stirnauflageabschnitt 201 sowohl in Richtung der hier angedeuteten Achsen X, Y einzustellen. Weiterhin ist es möglich, das Leitungsdurchführungselement 204 a auch geringfügig um dessen Längsachse Z zu schwenken.

In Figur 26 ist in Form einer Detaildarstellung die bei dem Ausführungsbeispiel nach den Figuren 24 und 25 verwirklichte Koppelungsstruktur zur Koppelung des Stirnauflageabschnitts 201 mit einem oberen Kopfband dargestellt. Im Bereich dieser Kopfband- Koppelungsstruktur ist das Bügelelement 220 in dem Stirnauflageabschnitt 201 verankert. Der Stirnauflageabschnitt 201 ist mit einer dünn auslaufenden Zunge 227 versehen, die sich in Applikationsposition des Stirnauflageabschnitts zwischen dem Bügelelement 220 und der Stirn des Maskenanwenders erstreckt und hierbei einen Kopfband-Umschlingungsbereich des Bügelelements 220 abpolstert und ein sanftes Abklingen der durch den Stirnauflageabschnitt 201 verursachten Flächenpressung unterstützt.

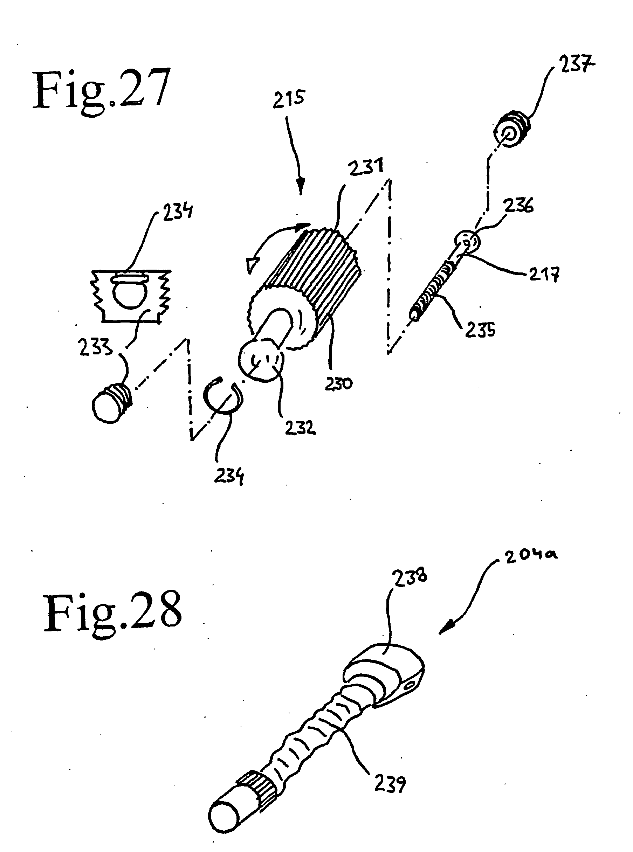

In Figur 27 ist eine bevorzugte Ausführungsform einer Stelltriebeinrichtung 215 dargestellt, wie sie bei dem Ausführungsbeispiel nach den Figuren 24 und 25 Anwendung finden kann. Die Stelltriebeinrichtung 215 umfasst die mit einer Außenumfangsrändelung 230 versehene Buchse 231, die eine Innenbohrung mit einer Innengewindestruktur aufweist. Die Buchse 231 umfasst einen Gelenkabschnitt 232, der hier kugelartig ausgebildet ist und drehbar in ein Sitzelement einsetzbar ist. Der Gelenkabschnitt 232 ist durch einen Federring 234 in dem Sitzelement 233 derart sicherbar, dass der Gelenkabschnitt 232 zwar drehbar und in einem hinreichenden Winkelbereich kippbewegbar, jedoch in axialer Richtung gesichert in dem Sitzelement 233 verankert ist. Ein Gewindeabschnitt 235 der Stellschraube 217 steht -im zusammengebauten Zustand- mit der Buchse 230 im Gewindeeingriff. An der Stellschraube 217 ist ein Fixierkopf 236 vorgesehen, der ebenfalls in ein Sitzelement 237 einsetzbar ist. Die Koppelung des Fixierkopfes 236 mit dem Sitzelement 237 ist abweichend von der Koppelung des Sitzelements 233 mit dem buchsenseitig vorgesehenen Gelenkabschnitt 232 derart bewerkstelligt, dass die Stellschraube 217 im wesentlichen undrehbar in dem Sitzelement 237 verankert ist.

Die hier gezeigte Stelltriebeinrichtung 215 ermöglicht es, den Abstand der beiden Sitzelemente 233, 237 durch individuelle Drehung der Buchse 231 stufenlos einzustellen.

Das bei dem Ausführungsbeispiel nach den Figuren 24 und 25 vorgesehene Leitungsdurchführungselement 204 a kann auch derart ausgebildet sein, dass dieses Teil der Atemgasleitung selbst bildet. Eine derartige Ausgestaltung des Leitungsdurchführungselements 204 a ist in Figur 28 dargestellt. Das Leitungsdurchführungselement 204 a umfasst den mit den Stelltriebeinrichtungen zusammenwirkenden Abstützabschnitt 238 und einen bei diesem Ausführungsbeispiel ausgebildeten, flexiblen Schlauchleitungsabschnitt 239. An dem Schlauchleitungsabschnitt 239 ist eine Steckbuchse vorgesehen, über die eine weitere Schlauchleitung drehbar anschließbar ist.

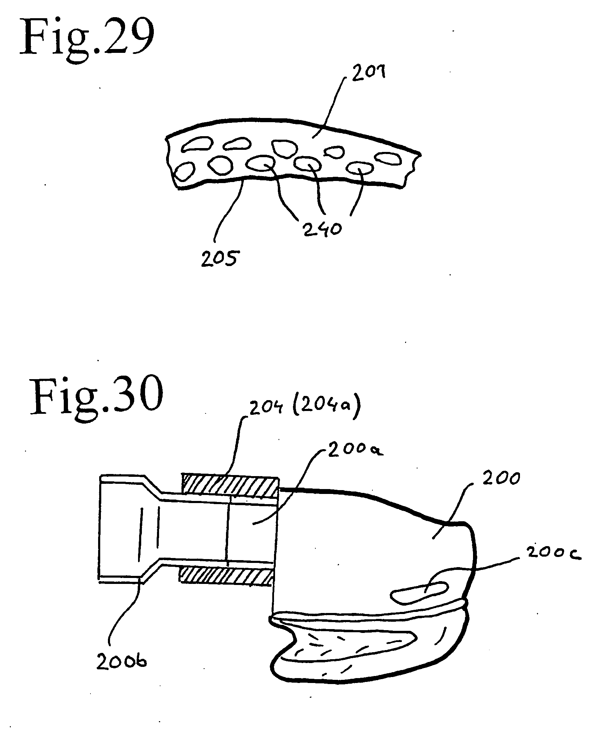

Figur 29 zeigt eine Detailansicht einer bevorzugten Ausführungsform des Stirnauflageabschnitts 201 im Bereich der, auf der Stirn des Maskenanwenders aufsitzenden Stirnauflagefläche 205. In dem Stirnauflageabschnitt 201 sind Kavernen 240 ausgebildet, durch welche eine besonders gleichmäßige Flächcnpressungsverteilung im Bereich der Auflagefläche 205 herbeiführbar ist.

Figur 30 zeigt in Form einer vereinfachten Schnittansicht wie das Leitungsverbindungselement 204 beziehungsweise auch das Leitungsdurchführungselement 204 a zur Koppelung eines seitens einer Atemmaske 200 vorgesehenen Anschlussstutzens 200 a mit einem weiteren Atemgasleitungsorgan 200 b verwendet werden kann. Bei diesem Ausführungsbeispiel bildet das Atemgasleitungsorgan 200 b Teil eines Auswaschventils wie es insbesondere in der Deutschen Patentanmeldung DE198.....beschrieben ist. An der Atemmaske 200 sind Bügelabschnitte 200 c vorgesehen, über welche die Atemmaske 200 mittels einer unteren Kopfbandanordnung im Nasenbereich des Maskenanwenders fixiert werden kann.

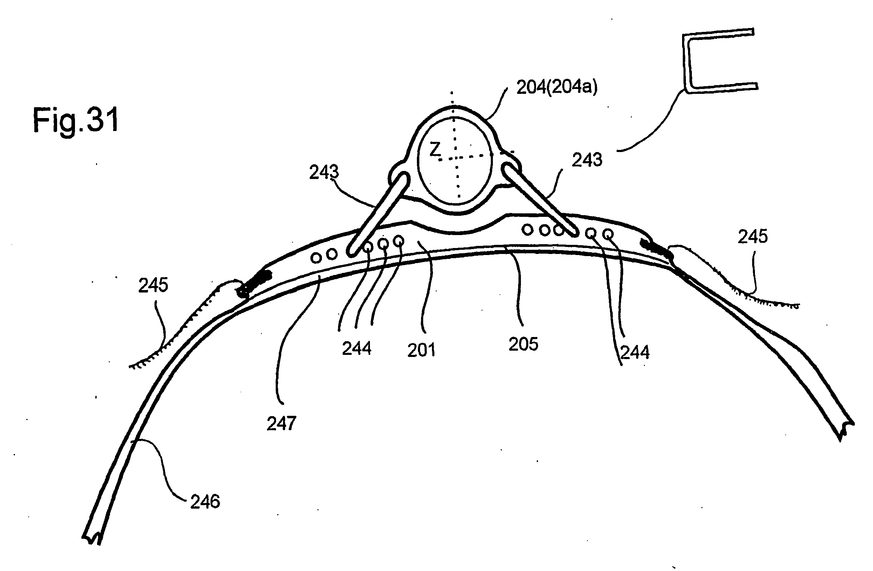

In Figur 31 ist eine Stirnauflageeinrichtung dargestellt, die weitgehende Gemeinsamkeiten mit der vorangehend bereits in Verbindung mit Figur 23 beschriebenen Ausführungsform aufweist. Das Leitungsverbindungselement 204 ( auch 204 a ) ist über Bügelelemente 243 mit dem Stirnauflageabschnitt 201 gekoppelt. Die Einstellung des Abstands der Zentralachse Z des Leitungsverbindungselements 204 relativ zur Unterseite ( Auflagefläche 205 ) des Stirnauflageabschnitts wird hierbei erreicht, indem die Bügelelemente 243 in entsprechend ausgewählte Bohrungen 244 einsteckbar sind. Je nachdem, welche Bohrungen 244 zur stirnauflageabschnittseitigen Verankerung der Bügelelemente 243 ausgewählt werden, ergeben sich hiermit unterschiedliche Abstände der Mittelachse Z gegenüber der Auflagefläche 205. Der Stirnauflageabschnitt 201 ist über Befestigungslaschen 245 an einer oberen Kopfbandanordnung 246 fixierbar. Die obere Kopfbandanordnung 246 umfasst bei diesem Ausführungsbeispiel einen Bandabschnitt 247, der sich zwischen den beiden Befestigungslaschen 245 unter dem Stirnauflageabschnitt 201 erstreckt und hierbei den Stirnauflageabschnitt zusätzlich auf der Stirn des Maskenanwenders abpolstert.

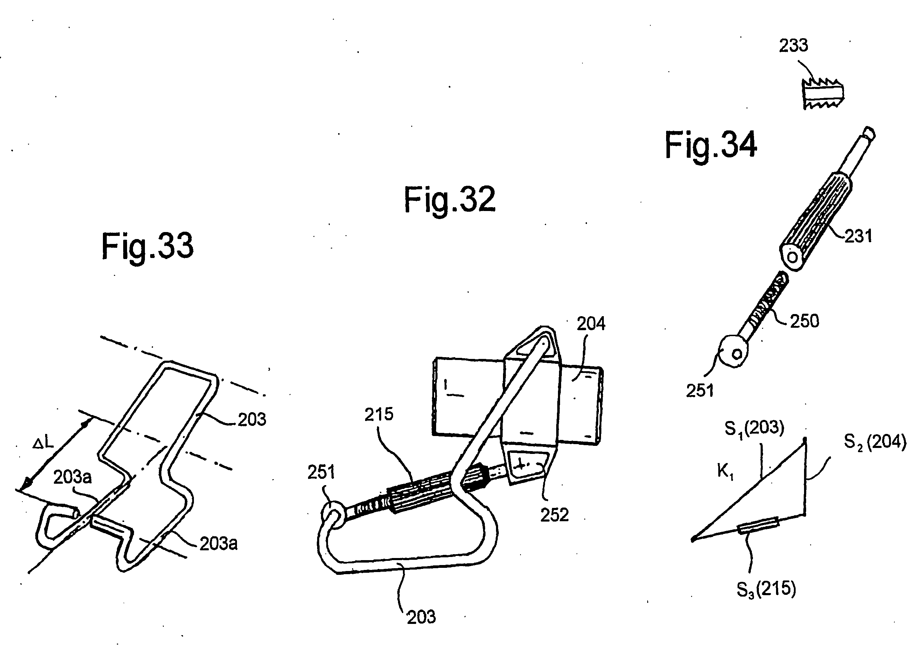

In Figur 32 ist eine Stirnabstützeinrichtung zur stirnseitigen Abstützung einer Atemmaskenanordnung dargestellt, die ebenfalls eine stufenlose Einstellung der Abstützhöhe ermöglicht Bei diesem Ausführungsbeispiel ist ein auf einen Anschlussstutzen einer Atemmaske aufsteckbares Leitungsverbindungselement 204 schwenkbewegbar mit der Koppelungsstruktur 203 gekoppelt. Die Koppelungsstruktur 203 ist bei diesem Ausführungsbeispiel als Drahtbiegeteil ausgebildet wie dies beispielhaft in Figur 33 dargestellt ist. Die Koppelungsstruktur 203 bildet im Zusammenspiel mit einer Stelltriebeinrichtung 215 das in der Skizze K 1 angedeutete Strebendreieck. Die Strebe S 1 wird hierbei durch die Koppelungsstruktur 203 gebildet, die Strebe S 2 wird durch das Leitungsverbindungselement 204 gebildet und die Strebe S 3 durch den Stelltrieb 215. Ein bevorzugter Aufbau des Stelltriebs 215 ist in Figur 34 dargestellt. Der Stelltrieb nach Figur 34 umfasst eine Gewindespindel 250, die über einen Gelenkkopf 251 wie in Figur 32 erkennbar mit der Koppelungsstruktur 203 schwenkbar, jedoch unverdrehbar gekoppelt ist. Die Gewindespindel 250 steht mit einer Innengewindebuchse 231 in Eingriff, die wiederum über ein Sitzelement 233 drehbar in einem radial austragenden Überstand 252 des Leitungsverbindungselements 204 verankert ist. Durch Drehung der Buchse 231 wird es möglich, den effektiven Abstand zwischen dem Gelenkkopf 251 und dem Sitzelement 233 zu verändern und damit die Koppelungsstruktur 203 relativ zu dem Leitungsverbindungselement 204 zu verschwenken. Hierdurch wird es möglich, unterschiedliche Relativpositionen der unteren Schenkel 203 a relativ zu einer an das Leitungsverbindungselement 204 angeschlossenen Atemmaske, und damit unterschiedliche Stirnabstände herbeizuführen.

In Figur 35 ist eine Atemmaskenanordnung dargestellt, die eine Atemmaske 200 und eine damit gekoppelte Stirnauflageeinrichtung aufweist. Die Stirnauflageeinrichtung umfasst einen elastisch verformbaren Leitungsabschnitt 260, der hier aus einem Elastomer-Material ausgebildet ist. Der Atemgasleitungsabschnitt 260 kann über eine Stelltriebeinrichtung 215 in einstellbarer Weise gekrümmt werden. Die Stelltriebeinrichtung umfasst bei diesem Ausführungsbeispiel eine Gewindeeinrichtung mit einer Innengewindebuchse 231, die mit einer Gewindespindel 250 in Eingriff steht. Durch Drehung der Buchse 230 ist es möglich, den Abstand zwischen den Punkten E 1 und E 2 zu verändern. Bei Veränderung dieses Abstands wird die hier erkennbare Koppelungsstruktur 203 elastisch verformt. Die Verformungscharakteristik der Koppelungsstruktur 203 ist derart gewählt, dass die an der Koppelungsstruktur 203 vorgesehene Polsterung 261 stets eine günstige Ausrichtung relativ zur Atemmaske 200 einnimmt. Die Stelltriebeinrichtung 215, der Atemgasleitungsabschnitt 260 und ein Schwenkbügelelement 262 bilden ein Strebendreieck, das in seinem Grundaufbau im wesentlichen den vorangehend in Verbindung mit den Figuren 32 - 34 und der darin enthaltenden Skizze K 1 beschriebenen Strebendreieck entspricht.

Die Polsterung 261 ist mit der Koppelungsstruktur 203 lösbar gekoppelt, sodass die Position der Polsterung in Richtung der hier angedeuteten Achse Z 2 einstellbar veränderbar ist.

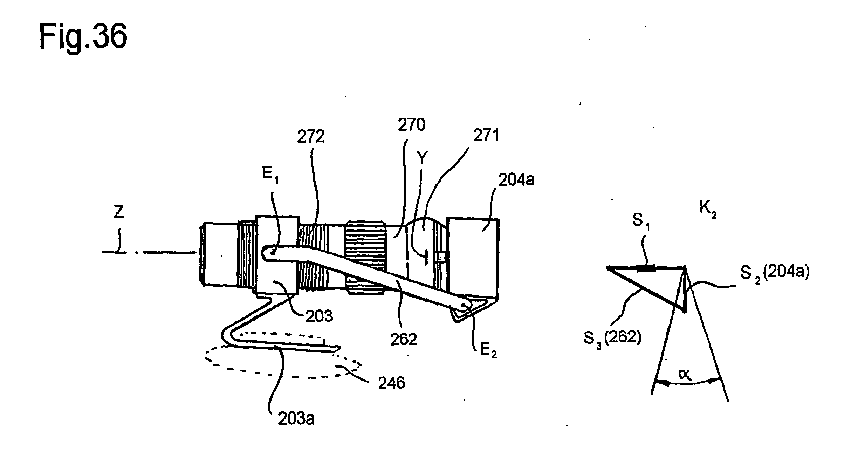

In Figur 36 ist eine Stirnabstützeinrichtung dargestellt, die ein auf eine Atemmaske 200 aufsteckbares Leitungsdurchführungselement 204 a aufweist. An diesem Leitungsdurchführungselement 204 a ist ein Kugelgelenkzapfen ausgebildet, der eine dreh- und schwenkbare Lagerung eines Rohrbuchsenelements 270 ermöglicht. Das Rohrbuchsenelement 270 umfasst hierzu einen Kalottenabschnitt 271, der derart ausgebildet ist, dass dieser im wesentlichen dicht sitzend, jedoch hinreichend leichtgängig bewegbar auf dem Zapfenabschnitt des Leitungsdurchführungselements 204 a sitzt. An dem Rohrhülsenelement 270 ist ein Außengewindeabschnitt 272 ausgebildet, der mit einer Koppelungsstruktur 203 in Gewindeeingriff steht. Die Koppelungsstruktur 203 umfasst Schenkelabschnitte 203 a, die mit einer vorzugsweise gepolsterten oberen Kopfbandanordnung 246 koppelbar sind. Durch Drehung des Rohrbuchsenelements 270 um dessen Zentralachse Z wird es aufgrund des Gewindeeingriffs zwischen dem Außengewindeabschnitt 272 und der Koppelungsstruktur 203 möglich, den Abstand zwischen den Punkten E 1 und E 2 stufenlos zu verändern. Im Bereich der Punkte E 1, E 2 ist ein Stellbügelelement 262 schwenkbewegbar mit der Koppelungsstruktur 203 bzw. dem Leitungsdurchführungselement 204 a gekoppelt. Das Rohrbuchsenelement 270 bildet bei diesem Ausführungsbeispiel gemeinsam mit der Koppelungsstruktur 203 eine Strebe S 1 in der hier dargestellten Skizze K 2. Das Leitungsdurchführungselement 204 a bildet mit seinem unmittelbar auf die Atemmaske aufsteckbaren Abschnitt die Strebe S 2 und das Schwenkbügelelement 262 bildet in diesem Dreieck die Strebe S 3. Durch Veränderung der wirksamen Länge der Strebe S 1 wird es möglich, das Rohrbuchsenelement 270 und gemeinsam mit diesem die Koppelungsstruktur 203 um die hier angedeutete Achse Y stufenlos zu schwenken.

Durch die vorangehend in Verbindung mit den Figuren 35 und 36 beschriebenen Stirnauflageeinrichtungen wird es möglich, die Abstützung einer Atemmaske im Bereich ihrer Nasenrückenauflage stufenlos auf die individuelle Gesichtstektur abzustimmen. Der Anpressdruck im Bereich des Nasenrückens kann insbesondere bei den beiden vorangehend beschriebenen Ausführungsformen verändert werden, ohne dass sich hierbei besondere Veränderungen durch die Stirnauflageeinrichtung bereitgestellten atemgasleitungsseitigen Anschlussabschnitts ergeben.

In Figur 37 ist eine Kopfbandanordnung dargestellt, die im Zusammenspiel mit den vorangehend beschriebenen Stirnabstützeinrichtungen und Atemmaskenanordnungen zur komfortablen Applikation einer Atemmaske Anwendung finden kann.

Die Anpressung der Atemmaske an das Gesicht des Anwenders kann entweder durch Aufbringung entsprechender Haltekräfte durch die obere Kopfbandanordnung 246 und eine untere Kopfbandanordnung 275 erfolgen. Es ist auch möglich, auf die untere Kopfbandanordnung 275 zu verzichten und insbesondere über die hier dargestellten Bügelelemente 276 das zur Generierung der Anpresskräfte im Bereich der Oberlippe erforderliche Drehmoment zu übertragen.

## Patentansprüche

1. Atemmaskenanordnung mit einem Gewölbekörper (2), einer Dichtlippeneinrichtung (1; 246) zur Auflage auf der Gesichtsfläche eines Maskenanwenders, einer Atemgasleitungseinrichtung (8, 9; 238, 239; 247, 248) zur Zuleitung von Atemgas zu einem von dem Gewölbekörper (2) begrenzten und mit der Nasen- und/oder Mundöffnung des Maskenanwenders in Verbindung stehenden Maskeninnenraum und einer Applikationsstruktur (3, 7, 11) zur Applikation der Dichtlippeneinrichtung (1; 246) gemeinsam mit dem Gewölbekörper (2), wobei die Applikationsstruktur (3, 7, 11) einen Trägerabschnitt (7; 204; 204a) aufweist, **dadurch gekennzeichnet, dass** an dem Trägerabschnitt ein Atemgasleitungsorgan (8; 200a, 200b) lösbar angekoppelt ist.

2. Atemmaskenanordnung nach Anspruch 1, **dadurch gekennzeichnet, dass** das Atemgasleitungsorgan (8; 200b) als Rohrstutzen ausgebildet ist.

3. Atemmaskenanordnung nach Anspruch 1 oder 2, **dadurch gekennzeichnet, dass** die Applikationsstruktur (3, 7, 11) einen Rahmenabschnitt (3) umfasst.

4. Atemmaskenanordnung nach Anspruch 3, **dadurch gekennzeichnet, dass** der Rahmenabschnitt (3) integral mit dem Trägerabschnitt (7; 204; 204a) ausgebildet ist.

5. Atemmaskenanordnung nach wenigstens einem der Ansprüche 3 bis 4, **dadurch gekennzeichnet, dass** der Trägerabschnitt (7; 204; 204a) im wesentlichen quer zum Rahmenabschnitt (3) ausgerichtet ist.

6. Atemmaskenanordnung nach wenigstens einem der Ansprüche 1 bis 5, **dadurch gekennzeichnet, dass** der Trägerabschnitt (7; 204; 204a) eine Einsatzöffnung aufweist und das Atemgasleitungsorgan (8; 200b) lösbar in diese Einsatzöffnung einsetzbar ist.

7. Atemmaskenanordnung nach wenigstens einem der Ansprüche 1 bis 6, **dadurch gekennzeichnet, dass** eine Verrastungseinrichtung (25) vorgesehen ist, zur Fixierung des Atemgasleitungsorganes (8; 200b) an dem Trägerabschnitt (7).

8. Atemmaskenanordnung nach Anspruch 7, **dadurch gekennzeichnet, dass** die Verrastungseinrichtung (25) als Bajonett- bzw. Drehverrastungseinrichtung ausgebildet ist.

9. Atemmaskenanordnung nach wenigstens einem der Ansprüche 1 bis 8, **dadurch gekennzeichnet, dass** der Gewölbekörper (2) abdichtend mit dem Atemgasleitungsorgan (8; 200b) gekoppelt ist.

10. Atemmaskenanordnung nach wenigstens einem der Ansprüche 1 bis 9, **dadurch gekennzeichnet, dass** der Gewölbekörper (2) aus einem Elastomermaterial gefertigt ist.

11. Atemmaskenanordnung nach wenigstens einem der Ansprüche 1 bis 10, **dadurch gekennzeichnet, dass** der Gewölbekörper (2) mit Öffnungen (6) versehen ist, zur Ableitung von CO2 befrachtetem Atemgas.

12. Atemmaskenanordnung nach wenigstens einem der Ansprüche 1 bis 11, **dadurch gekennzeichnet, dass** die Applikationsstruktur (3, 7, 11) eine Stirnabstützeinrichtung umfasst, zur Abstützung der Anordnung im Stirnbereich.

## Claims

1. A breathing mask arrangement comprising an arch body (2), a sealing lip arrangement (1; 246) for resting on the face area of a mask user, a respiratory gas supply means (8, 9; 238, 239; 247, 248) for supplying respiratory gas to an interior of the mask being limited by the arch body (2) and communicating with the nose and/or mouth opening of the mask user, and an application structure (3, 7, 11) for applying the sealing lip means (1; 246) together with the arch body (2), wherein the application structure (3, 7, 11) comprises a carrier portion (7; 204; 204a), **characterized in that** a respiratory gas supply element (8; 200a, 200b) is removably coupled to the carrier portion.

2. The breathing mask arrangement according to claim 1, **characterized in that** the respiratory gas supply element (8; 200b) is realized as a tube connection.

3. The breathing mask arrangement according to claim 1 or 2, **characterized in that** the application structure (3, 7, 11) comprises a frame portion (3).

4. The breathing mask arrangement according to claim 3, **characterized in that** the frame portion (3) is formed integrally with the carrier portion (7; 204; 204a).

5. The breathing mask arrangement according to at least one of claims 3 to 4, **characterized in that** the carrier portion (7; 204; 204a) is aligned essentially transversely with respect to the frame portion (3).

6. The breathing mask arrangement according to at least one of claims 1 to 5, **characterized in that** the carrier portion (7; 204; 204a) comprises an insert opening and the respiratory gas supply element (8; 200b) can be inserted removably into said insert opening.

7. The breathing mask arrangement according to at least one of claims 1 to 6, **characterized in that** a locking means (25) is provided for fixing the respiratory gas supply element (8; 200b) to the carrier portion (7).

8. The breathing mask arrangement according to claim 7, **characterized in that** the locking means (25) is configured as a bayonet or rotational locking means.

9. The breathing mask arrangement according to at least one of claims 1 to 8, **characterized in that** the arch body (2) is sealingly coupled with the respiratory gas supply element (8; 200b).

10. The breathing mask arrangement according to at least one of claims 1 to 9, **characterized in that** the arch body (2) is made of an elastomeric material.

11. The breathing mask arrangement according to at least one of claims 1 to 10, **characterized in that** the arch body (2) comprises openings (6) for dissipating CO₂-laden respiratory gas.

12. The breathing mask arrangement according to at least one of claims 1 to 11, **characterized in that** the application structure (3, 7, 11) comprises a forehead support means for supporting the arrangement in the forehead region.

## Revendications

1. Agencement formant masque respiratoire, comprenant un corps voûté (2), un système à lèvre d'étanchéité (1 ; 246) pour s'appliquer sur la surface du visage d'un utilisateur du masque, un système de conduits (8, 9 ; 238, 239 ; 247, 248) de gaz respiratoire pour l'amenée d'un gaz respiratoire au volume intérieur du masque, délimité par le corps voûté (2) et en communication avec l'ouverture du nez et/ou de la bouche de l'utilisateur du masque, et une structure d'application (3, 7, 11) pour l'application du système à lèvre d'étanchéité (1 ; 246) conjointement avec le corps voûté (2), ladite structure d'application (3, 7, 11) comprenant une portion porteuse (7 ; 204 ; 204a), **caractérisé en ce qu'**un organe de guidage (8 ; 200a, 200b) pour gaz respiratoire est couplé de façon détachable à la portion porteuse.

2. Agencement formant masque respiratoire selon la revendication 1, **caractérisé en ce que** l'organe de guidage (8 ; 200b) pour gaz respiratoire est réalisé sous forme d'une pipe tubulaire.

3. Agencement formant masque respiratoire selon la revendication 1 ou 2, **caractérisé en ce que** la structure d'application (3, 7, 11) comprend une portion en cadre (3).

4. Agencement formant masque respiratoire selon la revendication 3, **caractérisé en ce que** la portion en cadre (3) est réalisée de manière intégrale avec la portion porteuse (7 ; 204 ; 204a).

5. Agencement formant masque respiratoire selon l'une au moins des revendications 3 à 4, **caractérisé en ce que** la portion porteuse (7 ; 204 ; 204a) est orientée sensiblement perpendiculairement à la portion en cadre (3).

6. Agencement formant masque respiratoire selon l'une au moins des revendications 1 à 5, **caractérisé en ce que** la portion porteuse (7 ; 204 ; 204a) comprend une ouverture d'insertion, et l'organe de guidage (8 ; 200b) pour gaz respiratoire est susceptible d'être inséré de manière détachable dans cette ouverture d'insertion.

7. Agencement formant masque respiratoire selon l'une au moins des revendications 1 à 6, **caractérisé en ce qu'**il est prévu un moyen d'enclenchement (25) pour la fixation de l'organe de guidage (8 ; 200b) pour gaz respiratoire sur la portion porteuse (7).

8. Agencement formant masque respiratoire selon la revendication 7, **caractérisé en ce que** le moyen d'enclenchement (25) et réalisé sous forme de moyen d'enclenchement à baïonnette ou rotatif.

9. Agencement formant masque respiratoire selon l'une au moins des revendications 1 à 8, **caractérisé en ce que** le corps voûté (2) est couplé avec étanchement à l'organe de guidage (8 ; 200b) pour gaz respiratoire.

10. Agencement formant masque respiratoire selon l'une au moins des revendications 1 à 9, **caractérisé en ce que** le corps voûté (2) est réalisé en matériau élastomère.

11. Agencement formant masque respiratoire selon l'une au moins des revendications 1 à 10, **caractérisé en ce que** le corps voûté (2) est pourvu d'ouvertures (6) pour l'évacuation de gaz respiratoire chargé de CO₂.

12. Agencement formant masque respiratoire selon l'une au moins des revendications 1 à 11, **caractérisé en ce que** la structure d'application (3, 7, 11) comprend un organe de soutien frontal, pour le soutien de l'agencement dans la région frontale.
